# FASCICULE DE BREVET EUROPEEN

(11) **EP 3 408 676 B1**
(45) Date de publication et mention de la délivrance du brevet: **06.11.2019**
(21) Numéro de dépôt: 17706555.4
(22) Date de dépôt: 20.01.2017
(51) Int. Cl.: G01N 35/04, G01N 35/00

(54) **SYSTÈME DE CONVOYAGE DE SUPPORTS POUR RÉCIPIENTS D'ÉCHANTILLONS DE LIQUIDE BIOLOGIQUE, ET SYSTÈME D'ANALYSE AUTOMATIQUE COMPRENANT UN TEL SYSTÈME DE CONVOYAGE**
FÖRDERSYSTEM FÜR HALTERUNGEN FÜR BEHÄLTER BIOLOGISCHER FLÜSSIGKEITSPROBEN UND AUTOMATISCHES ANALYSESYSTEM MIT SOLCH EINEM FÖRDERSYSTEM
SYSTEM FOR CONVEYING HOLDERS FOR CONTAINERS FOR BIOLOGICAL LIQUID SAMPLES, AND AUTOMATIC ANALYSIS SYSTEM COMPRISING SUCH A CONVEYING SYSTEM

(30) Priorité: 25.01.2016 FR 1650553
(43) Date de publication de la demande: 05.12.2018
(73) Titulaire: ARTEION, 75007 Paris (FR)
(72) Inventeur: ROUSSEAU, Alain, 75001 Paris (FR)
(74) Mandataire: Verriest, Philippe
(86) Numéro de dépôt international: PCT/FR2017/050123
(87) Numéro de publication internationale: WO 2017/129882

(56) Documents cités:
- US-A1- 2003 044 319
- US-A1- 2004 134 750
- US-A1- 2015 014 125

## Description

La présente invention concerne un système de convoyage configuré pour convoyer des supports de récipients destinés à supporter des récipients contenant des échantillons de liquide biologique, et un système d'analyse automatique pour diagnostic in vitro comprenant un tel système de convoyage.

Un système d'analyse automatique pour diagnostic in vitro, également nommé système de laboratoire automatique, comprend de façon connue :
- une pluralité de supports de récipients destinés à supporter des récipients contenant des échantillons de liquide biologique à analyser,
- un système de convoyage comprenant :
   - une unité de convoyage configurée pour recevoir et convoyer des supports de récipients selon un chemin de convoyage, l'unité de convoyage comportant généralement une courroie de convoyage ou un dispositif de convoyage magnétique,
   - un zone de chargement configurée pour charger des supports de récipients sur l'unité de convoyage, et
- une pluralité de postes d'analyse et/ou de mesure disposés le long du chemin de convoyage, et destinés à être alimentés en supports de récipient par l'unité de convoyage.

Bien qu'un tel système d'analyse automatique limite grandement les manipulations fastidieuses pour un opérateur, il n'assure pas une capacité de convoyage élevée et une cadence d'analyse élevée, notamment lorsqu'un support de récipient doit être convoyé entre différents postes d'analyse et/ou de mesure.

En outre, la majorité des systèmes de convoyage connus présente une grande complexité du fait en particulier de la présence de courroies de convoyage ou de dispositifs de convoyage magnétiques, et cette complexité est généralement répartie sur l'intégralité des systèmes de convoyage. Les coûts des systèmes de convoyage connus sont donc élevés, et parfois supérieurs aux coûts des postes d'analyses et/ou de mesure. Une telle complexité des systèmes de convoyage induit également des configurations et des installations laborieuses et une maintenabilité délicate, et susceptible de causer de nombreuses défaillances potentielles.

De plus, en cas de défaillance de l'unité de convoyage, par exemple en cas de défaillance du mécanisme d'entraînement de la courroie de convoyage ou d'une usure de la courroie de convoyage, la durée d'immobilisation du système d'analyse automatique peut s'avérer longue et coûteuse pour un laboratoire d'analyse.

Le document US2015/014125 divulgue un système de convoyage configuré pour convoyer des supports de récipients destinés à supporter des récipients contenant des échantillons de liquide biologique, le système de convoyage comportant un chariot de convoyage autopropulsé, déplaçable le long d'un chemin de convoyage, configuré pour déplacer en translation le support de récipients.

La présente invention vise à remédier à ces inconvénients.

Le problème technique à la base de l'invention consiste donc à fournir un système de convoyage qui soit de structure simple, économique et fiable, tout en assurant une capacité de convoyage élevée, une cadence d'analyse optimisée et une maintenabilité simple et rapide.

A cet effet, la présente invention concerne un système de convoyage configuré pour convoyer des supports de récipients destinés à supporter des récipients contenant des échantillons de liquide biologique, le système de convoyage comportant au moins :
- un élément de guidage de support définissant un chemin de guidage, l'élément de guidage de support étant configuré pour recevoir un support de récipients et guider en translation ledit support de récipients le long du chemin de guidage,
- un chariot de convoyage autopropulsé déplaçable le long d'un chemin de convoyage s'étendant le long de l'élément de guidage de support, le chariot de convoyage autopropulsé comprenant un élément d'entraînement monté mobile entre au moins une position d'entraînement dans laquelle l'élément d'entraînement est configuré pour transmettre un mouvement d'entraînement au support de récipients reçu sur l'élément de guidage de support, et une position de libération dans laquelle l'élément d'entraînement est configuré pour libérer le support de récipients, le chariot de convoyage autopropulsé étant configuré pour déplacer en translation le support de récipients le long du chemin de guidage lorsque l'élément d'entraînement est dans la position d'entraînement et le chariot de convoyage autopropulsé se déplace le long du chemin de convoyage.

Une telle configuration du système de convoyage, et plus particulièrement du chariot de convoyage, permet un convoyage simple, aisé et rapide d'un support de récipients entre des zones de chargement et de déchargement et des postes d'analyse et de mesure, et assure ainsi une cadence de convoyage et d'analyse élevée.

De plus, en cas de défaillance du chariot de convoyage, il suffit de remplacer ce dernier par un autre chariot de convoyage, ce qui assure une courte durée d'immobilisation du système de convoyage, et limite donc grandement les pertes financières pour le laboratoire d'analyse.

En outre, puisque le chariot de convoyage est autopropulsé, les chemins de guidage et de convoyage peuvent être essentiellement « passifs », et donc être définis par des éléments simples, tels que des glissières de guidage, ce qui simplifie grandement le système de convoyage selon la présente invention et augmente la fiabilité d'un tel système, en réduit les coûts et permet une installation plus rapide et aisée.

Le système de convoyage peut en outre présenter une ou plusieurs des caractéristiques suivantes, prises seules ou en combinaison.

Selon un mode de réalisation de l'invention, le système de convoyage est un système de convoyage pour système d'analyse automatique pour diagnostic in vitro. Avantageusement, le système de convoyage est configuré pour convoyer des supports de récipients vers au moins un poste d'analyse et/ou de mesure.

Selon un mode de réalisation de l'invention, en position d'entraînement, l'élément d'entraînement est configuré pour être solidarisé en translation au support de récipients reçu sur l'élément de guidage de support.

Selon un mode de réalisation de l'invention, l'élément de guidage de support est configuré pour recevoir et guider en translation au moins une embase du support de récipients.

Selon un mode de réalisation de l'invention, le chariot de convoyage autopropulsé est déplaçable le long d'un chemin de convoyage dans un premier sens de déplacement et dans un deuxième sens de déplacement opposé au premier sens de déplacement.

Selon un mode de réalisation de l'invention, le chemin de guidage est sensiblement rectiligne.

Selon un mode de réalisation de l'invention, le chemin de convoyage est sensiblement parallèle au chemin de guidage.

Selon un mode de réalisation de l'invention, l'élément de guidage de support est agencé pour guider en translation le support de récipients sensiblement parallèlement à une direction d'extension du support de récipients.

Selon un mode de réalisation de l'invention, l'élément de guidage de support comporte une première surface de guidage latérale configurée pour coopérer avec une première surface latérale du support de récipients, et une deuxième surface de guidage latérale configurée pour coopérer avec une deuxième surface latérale du support de récipients opposée à la première surface latérale.

Selon un mode de réalisation de l'invention, le chariot de convoyage autopropulsé et l'élément de guidage de support sont agencés pour maintenir le support de récipients sensiblement vertical lors de ses déplacements le long du chemin de guidage.

Selon un mode de réalisation de l'invention, l'élément d'entraînement est monté pivotant autour d'un axe de pivotement et entre la position d'entraînement et la position de libération.

Selon un mode de réalisation de l'invention, l'élément d'entraînement comporte deux branches d'entraînement espacées l'une de l'autre et configurées pour coopérer avec le support de récipients lorsque l'élément d'entraînement est dans sa position d'entraînement. Avantageusement, les deux branches d'entraînement sont espacées l'une de l'autre d'une distance correspondant sensiblement à la longueur du support de récipients.

Selon un mode de réalisation de l'invention, les deux branches d'entraînement sont configurées pour coopérer respectivement avec des parois latérales opposées du support de récipients.

Selon un mode de réalisation de l'invention, chaque branche d'entraînement comporte un doigt d'accrochage configuré pour être inséré dans une encoche d'accrochage respective ménagée sur le support de récipients.

Selon un mode de réalisation de l'invention, l'axe de pivotement s'étend sensiblement parallèlement au chemin de convoyage.

Selon un mode de réalisation de l'invention, le chariot de convoyage autopropulsé comporte un dispositif d'actionnement configuré pour déplacer l'élément d'entraînement entre les positions d'entraînement et de libération. Selon un mode de réalisation de l'invention, le dispositif d'actionnement est configuré pour faire pivoter l'élément d'entraînement autour de son axe de pivotement. Le dispositif d'actionnement peut comporter différents types d'actionneurs, tels qu'un moteur couplé en rotation à l'élément d'entraînement.

Selon un mode de réalisation de l'invention, le chariot de convoyage autopropulsé comporte au moins une roue motrice, et au moins un mécanisme d'entraînement en rotation configuré pour entraîner en rotation l'au moins une roue motrice. L'au moins un mécanisme d'entraînement en rotation comporte par exemple un moteur d'entraînement couplé en rotation à l'au moins un roue motrice.

Selon un mode de réalisation de l'invention, le chariot de convoyage autopropulsé comporte un corps de chariot sur lequel est montée l'au moins une roue motrice.

Selon un mode de réalisation de l'invention, le chariot de convoyage autopropulsé comporte deux roues motrices. Les deux roues motrices peuvent par exemple être motorisées indépendamment l'une de l'autre. A cet effet, le chariot de convoyage autopropulsé peut comporter deux mécanismes d'entraînement en rotation configurés pour entraîner en rotation chacun une roue motrice respective.

Selon un mode de réalisation de l'invention, le système de convoyage comporte un élément de guidage de chariot définissant le chemin de convoyage, l'élément de guidage de chariot étant configuré pour recevoir et guider le chariot de convoyage autopropulsé lors des déplacements du chariot de convoyage autopropulsé le long du chemin de convoyage.

Selon un mode de réalisation de l'invention, l'élément de guidage de support est une glissière de guidage de support.

Selon un mode de réalisation de l'invention, l'élément de guidage de chariot est une glissière de guidage de chariot.

Selon un mode de réalisation de l'invention, le chariot de convoyage autopropulsé comporte des galets de guidage configurés pour coopérer avec l'élément de guidage de chariot lors des déplacements du chariot de convoyage autopropulsé le long du chemin de convoyage.

Selon un mode de réalisation de l'invention, le système de convoyage comprend au moins une zone de prélèvement ou de transfert, également nommée zone de réception, disposée le long du chemin de guidage et à l'extérieur du chemin de guidage, le chariot de le chariot de convoyage autopropulsé étant configuré pour déplacer le support de récipients reçu sur l'élément de guidage de support dans l'au moins une zone de prélèvement ou de transfert de manière à libérer le chemin de guidage.

Selon un mode de réalisation de l'invention, l'au moins une zone de prélèvement ou de transfert est destinée à être disposée à proximité d'un poste de traitement d'échantillons.

Selon un mode de réalisation de l'invention, l'au moins une zone de prélèvement ou de transfert comporte un emplacement de prélèvement agencé pour recevoir et stocker au moins temporairement le support de récipients.

Selon un mode de réalisation de l'invention, le système de convoyage comporte des moyens de détection agencés pour détecter la réception d'un support de récipients dans l'au moins une zone de prélèvement ou de transfert.

Selon un mode de réalisation de l'invention, le système de convoyage comporte au moins un repère de positionnement disposé sur le chemin de convoyage, et le chariot de convoyage autopropulsé comporte des moyens de détection, tels qu'un lecteur optique, un détecteur RFID ou inductif, agencés pour détecter l'au moins repère de positionnement, et des moyens de commande, tels qu'un circuit intégré ou un microprocesseur, agencés pour commander l'immobilisation du chariot de convoyage autopropulsé lorsque les moyens de détection détectent l'au moins un repère de positionnement. Le système de convoyage comporte par exemple au moins un repère de positionnement disposé en regard de l'au moins une zone de prélèvement ou de transfert.

Selon un mode de réalisation de l'invention, l'au moins un repère de positionnement peut être formé par une barrière optique, un code-barres, un code QR ou encore une étiquette RFID disposé(e) sur le chemin de convoyage.

Selon un mode de réalisation de l'invention, l'élément de guidage de support comporte une ouverture de passage débouchant dans l'au moins une zone de prélèvement ou de transfert et destinée au passage d'un support de récipients.

Selon un mode de réalisation de l'invention, le chariot de convoyage autopropulsé comporte un corps de chariot et un élément de support sur lequel est monté mobile l'élément d'entraînement, l'élément de support étant monté mobile en translation par rapport au corps de chariot selon une direction de déplacement transversale au chemin de convoyage et entre au moins une position de convoyage et une position de dégagement.

Selon un mode de réalisation de l'invention, l'élément de support et l'élément d'entraînement sont configurés de telle sorte que, lorsque le chariot de convoyage autopropulsé est disposé en regard de l'au moins une zone de prélèvement ou de transfert et l'élément d'entraînement est dans la position d'entraînement, un déplacement de l'élément de support de la position de convoyage à la position de dégagement peut entraîner un déplacement du support de récipients du chemin de guidage à l'au moins une zone de prélèvement ou de transfert.

Selon un mode de réalisation de l'invention, l'élément de support comporte une surface de poussée configurée pour exercer un effort de poussée contre le support de récipients lorsque le support de récipients est reçu sur l'élément de guidage de support et l'élément de support est déplacé vers la position de dégagement. La surface de poussée est par exemple configurée pour prendre appuis contre une surface latérale du support de récipients.

Selon un mode de réalisation de l'invention, l'élément de support et l'élément d'entraînement sont configurés de telle sorte que le chariot de convoyage autopropulsé est apte à déplacer le support de récipients de la zone de prélèvement ou de transfert au chemin de guidage.

Avantageusement, l'élément de support et l'élément d'entraînement sont configurés de telle sorte que, lorsque le chariot de convoyage autopropulsé est disposé en regard de l'au moins une zone de prélèvement ou de transfert et l'élément d'entraînement est dans la position d'entraînement, un déplacement de l'élément de support de la position de dégagement à la position de convoyage peut entraîner un déplacement du support de récipients de la zone de prélèvement ou de transfert au chemin de guidage.

Selon un mode de réalisation de l'invention, le chariot de convoyage autopropulsé comporte un mécanisme d'entraînement en translation configuré pour déplacer en translation l'élément de support par rapport au corps de chariot. Le mécanisme d'entraînement en translation peut comporter différents types d'actionneurs, et peut par exemple comporter un vérin comprenant une première portion reliée à l'élément de support et une deuxième portion reliée au corps de chariot. Selon une variante de réalisation, le mécanisme d'entraînement en translation peut comporter une crémaillère prévue sur l'élément de support, et une roue dentée prévue sur le corps de chariot et configurée pour coopérer avec la crémaillère.

Selon un mode de réalisation de l'invention, le chariot de convoyage autopropulsé comporte une batterie configurée pour alimenter électriquement le chariot de convoyage autopropulsé. Avantageusement, la batterie est rechargeable. Par exemple, la batterie peut être rechargée par contact ou par induction.

Selon un mode de réalisation de l'invention, la batterie est configurée pour alimenter électriquement le mécanisme d'entraînement en translation, le mécanisme d'entraînement en rotation et/ou le dispositif d'actionnement.

Selon un mode de réalisation de l'invention, le système de convoyage comporte une zone de rechargement comportant un dispositif de rechargement électrique configuré pour recharger électriquement la batterie lorsque le chariot de convoyage autopropulsé est situé dans la zone de rechargement.

Selon un mode de réalisation de l'invention, le système de convoyage comprend une zone de chargement destinée à stocker le support de récipients et comprenant un dispositif de chargement agencé pour charger le support de récipients, stocké dans le zone de chargement, dans le chemin de guidage défini par l'élément de guidage de support, et une zone de déchargement dans laquelle le support de récipients est destiné à être déchargé.

Selon un mode de réalisation de l'invention, le système de convoyage comporte un repère de positionnement disposé en regard de la zone de chargement et un repère de positionnement disposé en regard de la zone de déchargement.

Selon un mode de réalisation de l'invention, le dispositif de chargement comporte un chariot de chargement autopropulsé déplaçable le long d'un chemin de chargement, le chariot de chargement autopropulsé comprenant un élément d'entraînement monté mobile entre une position d'entraînement dans laquelle l'élément d'entraînement est configuré pour transmettre un mouvement d'entraînement à l'au moins un support de récipients stocké dans la zone de chargement, et une position de libération dans laquelle l'élément d'entraînement est configuré pour libérer ledit support de récipients, le chariot de chargement autopropulsé étant configuré pour déplacer en translation ledit support de récipients le long du chemin de chargement et pour insérer ledit support de récipients sur l'élément de guidage de support lorsque le chariot de chargement autopropulsé se déplace le long du chemin de chargement et lorsque l'élément d'entraînement est dans la position d'entraînement.

Selon un mode de réalisation de l'invention, le chemin de chargement est transversal au chemin de guidage.

Selon un mode de réalisation de l'invention, l'élément d'entraînement est monté mobile en translation entre la position d'entraînement et la position de libération, par exemple selon une direction de déplacement sensiblement verticale.

Selon un mode de réalisation de l'invention, l'élément d'entraînement est configuré pour saisir ou accrocher le support de récipients lorsque l'élément d'entraînement est dans la position d'entraînement.

Selon un mode de réalisation de l'invention, le chariot de convoyage autopropulsé est configuré pour déplacer un support de récipients, reçu dans l'élément de guidage de support, dans la zone de déchargement.

Selon un mode de réalisation de l'invention, la zone de chargement comporte des premiers moyens de guidage agencés pour guider en translation un support de récipients stocké dans la zone de chargement selon une direction de chargement. Les premiers moyens de guidage peuvent par exemple comporter un rail de guidage.

Selon un mode de réalisation de l'invention, la zone de déchargement comporte des seconds moyens de guidage agencés pour guider en translation un support de récipients déchargé dans la zone de déchargement selon une direction de déchargement. Les seconds moyens de guidage peuvent par exemple comporter un rail de guidage.

Selon un mode de réalisation de l'invention, les premiers et/ou seconds moyens de guidage sont agencés pour guider en translation un support de récipients sensiblement perpendiculairement à la direction d'extension du support de récipients.

Selon un mode de réalisation de l'invention, les premiers et/ou seconds moyens de guidage sont configurés pour coopérer avec des moyens de guidage complémentaires prévus sur chaque support de récipients, et plus particulièrement sur l'embase de chaque support de récipients. Par exemple, les premiers et/ou seconds moyens de guidage sont configurés pour coopérer avec une encoche de guidage de forme complémentaire prévue dans l'embase de chaque support de récipients.

Selon un mode de réalisation de l'invention, le système de convoyage comporte un dispositif de lecture de code d'identification configuré pour lire optiquement des codes d'identification portés par des récipients supportés par le support de récipients lorsque le support de récipients est reçu sur l'élément de guidage de support. Les informations enregistrées dans chaque code d'identification peuvent comporter par exemple le numéro de référence de l'échantillon respectif, qui est relié de manière univoque au le nom du donneur de l'échantillon respectif.

Selon un mode de réalisation de l'invention, le dispositif de lecture de code d'identification est monté mobile en translation selon une direction de déplacement sensiblement parallèle au chemin de convoyage.

Selon un mode de réalisation de l'invention, les codes d'identification portés par les récipients sont formés par des codes-barres, par exemple monodimensionnels ou bidimensionnels, ou des codes QR disposés sur la surface extérieure des récipients.

Selon un mode de réalisation de l'invention, le système de convoyage comprend un module d'entraînement en rotation configuré pour entraîner en rotation des récipients supportés par le support de récipients lorsque le support de récipients est reçu sur l'élément de guidage de support, de manière à permettre la lecture des codes d'identification portés par lesdits récipients par le dispositif de lecture de code d'identification.

Selon un mode de réalisation de l'invention, le module d'entraînement en rotation comporte un organe d'actionnement monté mobile en rotation autour d'un axe vertical, et agencé pour entraîner en rotation un récipient supporté par le support de récipients autour d'un axe d'extension dudit récipient.

Selon un mode de réalisation de l'invention, le module d'entraînement en rotation comprend des premiers moyens de déplacement agencés pour déplacer en translation l'organe d'actionnement suivant une première direction de déplacement sensiblement parallèle au chemin de convoyage, et des seconds moyens de déplacement agencés pour déplacer en translation l'organe d'actionnement suivant une seconde direction de déplacement sensiblement verticale.

Selon un mode de réalisation de l'invention, le système de convoyage comporte une unité de commande configurée pour communiquer à distance avec le chariot de convoyage autopropulsé. L'unité de commande peut être un ordinateur, par exemple de type PC.

Selon un mode de réalisation de l'invention, l'unité de commande est configurée pour communiquer sans fil, par exemple par wifi ou Bluetooth, avec le chariot de convoyage autopropulsé.

Selon un mode de réalisation de l'invention, le système de convoyage comporte un rotor de stockage d'axe de rotation sensiblement vertical, le rotor de stockage comportant une pluralité de logements de stockage configurés chacun pour recevoir un support de récipients provenant du chemin de guidage. Un tel rotor de stockage permet de stocker temporairement des supports de récipients, et de pouvoir les réinsérer dans le premier ou le deuxième chemin de guidage en vue éventuellement d'un convoyage vers un poste d'analyse et/ou de mesure avant leur déchargement.

Selon un mode de réalisation de l'invention, le système de convoyage comprend des moyens d'entraînement en rotation associés au rotor de stockage et agencés pour entraîner en rotation le rotor de stockage autour de son axe de rotation.

Selon un mode de réalisation de l'invention, les moyens d'entraînement en rotation associés au rotor de stockage sont agencés pour entraîner en rotation le rotor de stockage dans un premier sens et dans un deuxième sens opposé au premier sens.

Selon un mode de réalisation de l'invention, le rotor de stockage est disposé à une extrémité du chemin de convoyage.

Selon un mode de réalisation de l'invention, le système de convoyage comporte un premier et un deuxième éléments de guidage de support définissant respectivement un premier et un deuxième chemins de guidage, les premier et deuxième éléments de guidage de support étant disposés de part et d'autre du chemin de convoyage, l'élément d'entraînement du chariot de convoyage autopropulsé étant monté mobile entre une première position d'entraînement dans laquelle l'élément d'entraînement est configuré pour transmettre un mouvement d'entraînement à un support de récipients reçu sur le premier élément de guidage de support, une deuxième position d'entraînement dans laquelle l'élément d'entraînement est configuré pour transmettre un mouvement d'entraînement à un support de récipients reçu sur le deuxième élément de guidage de support, et une position de libération dans laquelle l'élément d'entraînement est configuré pour libérer lesdits supports de récipients.

Avantageusement, le chariot de convoyage autopropulsé est configuré pour déplacer en translation le support de récipients reçu sur le premier élément de guidage de support le long du premier chemin de guidage lorsque le chariot de convoyage autopropulsé se déplace le long du chemin de convoyage et lorsque l'élément d'entraînement est dans la première position d'entraînement, et pour déplacer en translation le support de récipients reçu sur le deuxième élément de guidage de support le long du deuxième chemin de guidage lorsque le chariot de convoyage autopropulsé se déplace le long du chemin de convoyage et lorsque l'élément d'entraînement est dans la deuxième position d'entraînement.

Avantageusement, l'élément de support est mobile entre une première position de dégagement et une deuxième position de dégagement, l'élément de support et l'élément d'entraînement étant configurés de telle sorte que, lorsque le chariot de convoyage autopropulsé est disposé en regard de la zone de déchargement et l'élément d'entraînement est dans la position d'entraînement, un déplacement de l'élément de support de la position de convoyage à la deuxième position de dégagement entraîne un déplacement du support de récipients du chemin de guidage à la zone de déchargement.

Selon un mode de réalisation de l'invention, le rotor de stockage est configuré pour permettre un transfert d'un support d'échantillon du premier élément de guidage de support au deuxième élément de guidage de support, et inversement.

Selon un mode de réalisation de l'invention, le système de convoyage comporte un dispositif de transfert configuré pour transférer un support de récipients du premier élément de guidage de support au deuxième élément de guidage de support, et inversement. Ces dispositions permettent un transfert aisé et rapide d'un support de récipients entre les premier et deuxième chemins de guidage.

Selon un mode de réalisation de l'invention, le dispositif de transfert comporte une partie de convoyage principale, une première et une deuxième parties de transfert disposées de part et d'autre de la partie de convoyage principale, et une première et une deuxième parties de convoyage secondaires disposées de part et d'autre des première et deuxième parties de transfert, le dispositif de transfert étant déplaçable entre une position de convoyage dans laquelle les première et deuxième parties de transfert définissent en partie respectivement les premier et deuxième chemins de guidage et la partie de convoyage principale définit en partie le chemin de convoyage, une première position de transfert dans laquelle la première partie de transfert et la première partie de convoyage secondaire définissent en partie respectivement le deuxième chemin de guidage et le chemin de convoyage, et une deuxième position de transfert dans laquelle la deuxième partie de transfert et la deuxième partie de convoyage secondaire définissent en partie respectivement le premier chemin de guidage et le chemin de convoyage.

Selon un mode de réalisation de l'invention, le dispositif de transfert est mobile en translation selon une direction de déplacement s'étendant transversalement au chemin de convoyage, et avantageusement sensiblement perpendiculairement au chemin de convoyage.

Selon un mode de réalisation de l'invention, le premier élément de guidage de support comporte une ouverture de chargement disposée en regard de la zone de chargement et destinée au passage d'un support de récipients, et le deuxième élément de guidage de support comporte une ouverture de déchargement disposée en regard de la zone de déchargement et destinée au passage d'un support de récipients.

Selon un mode de réalisation de l'invention, le système de convoyage comporte au moins un support de récipients destiné à supporter des récipients.

Selon un mode de réalisation de l'invention, l'au moins un support de récipients s'entend selon direction d'extension. Avantageusement, l'au moins un support de récipients présente une forme générale parallélépipédique.

Selon un mode de réalisation de l'invention, l'au moins un support de récipients comporte une pluralité de logements de réception, par exemple cylindriques, alignés selon la direction d'extension de l'au moins un support de récipients. Avantageusement, chaque logement de réception est ouvert vers le haut.

Selon un mode de réalisation de l'invention, l'au moins un support de récipients comprend des fenêtres de lecture permettant une lecture optique des codes d'identification portés par les récipients reçus sur le support de récipients. Avantageusement, chaque fenêtre de lecture débouche dans un logement de réception respectif.

Selon un mode de réalisation de l'invention, l'au moins un support de récipients est configuré pour supporter des tubes d'échantillons.

La présente invention concernant en outre un système d'analyse automatique pour diagnostic in vitro, comprenant un système de convoyage selon l'invention, et au moins un poste de traitement d'échantillons, tel qu'un poste d'analyse et/ou de mesure, disposé le long du chemin de guidage.

Selon un mode de réalisation de l'invention, l'au moins un poste de traitement d'échantillons est disposé à proximité de l'au moins une zone de prélèvement ou de transfert.

Selon un mode de réalisation de l'invention, l'au moins un poste de traitement d'échantillons est un poste d'analyse et/ou de mesure pour diagnostic in vitro, et plus particulièrement pour réaliser des tests sanguins, tels que des tests sur sang total.

Selon un mode de réalisation de l'invention, l'au moins un poste de traitement d'échantillons comporte au moins un module parmi un module de lecture spectrophotométrique, un module de lecture par fluorescence, un module de lecture par luminescence, un module de mesure de coagulation.

Selon un mode de réalisation de l'invention, l'au moins un poste de traitement d'échantillons comporte un dispositif d'agitation de support, et un dispositif de transfert configuré pour transférer un support de récipients entre la zone de prélèvement ou de transfert et le dispositif d'agitation.

Selon un mode de réalisation de l'invention, l'au moins un poste de traitement d'échantillons comporte un dispositif d'analyse tel que décrit dans le document FR2998057.

Selon un mode de réalisation de l'invention, l'au moins un poste de traitement d'échantillons comporte un dispositif de prélèvement configuré pour prélever des échantillons dans les récipients supportés par un support de récipients, et par exemple dans les récipients supportés par un support de récipients reçu dans l'au moins une zone de prélèvement ou de transfert.

Selon un mode de réalisation de l'invention, le dispositif de prélèvement comporte une tête de prélèvement équipée d'une aiguille de prélèvement, des premiers moyens de déplacement agencés pour déplacer la tête de prélèvement en translation suivant une direction sensiblement horizontale et sensiblement parallèle au chemin de guidage, et des seconds moyens de déplacement agencés pour déplacer la tête de prélèvement suivant une direction sensiblement verticale.

Selon un mode de réalisation de l'invention, l'au moins un poste de traitement d'échantillons comporte des moyens de détection agencés pour détecter la réception d'un support de récipients dans l'au moins une zone de prélèvement ou de transfert.

Selon un mode de réalisation de l'invention, le système d'analyse automatique comporte une pluralité de postes de traitement d'échantillons disposés le long du chemin de guidage.

De toute façon l'invention sera bien comprise à l'aide de la description qui suit en référence au dessin schématique annexé représentant, à titre d'exemple non limitatif, une forme d'exécution de ce système de convoyage.
Figure 1 est une vue en perspective d'un système d'analyse automatique pour diagnostic in vitro selon l'invention.
Figures 2 et 3 sont des vues en perspective d'un chariot de convoyage appartenant au système d'analyse.
Figure 4 est une vue en perspective d'un support de récipient appartenant au système d'analyse et retenu par le chariot de convoyage de la figure 2.
Figure 5 est une vue en perspective du support de récipient de la figure 4.
Figures 6 à 9 sont des vues partielles en perspective du système d'analyse montrant le chariot de convoyage dans différentes positions de fonctionnement.
Figure 10 est une vue partielle en perspective d'une zone de chargement appartenant au système d'analyse.
Figure 11 est une vue en perspective montrant plus particulièrement un chariot de chargement appartenant à la zone de chargement.
Figure 12 est une vue en perspective montrant plus particulièrement un module d'entraînement en rotation appartenant au système d'analyse.
Figure 13 est une vue en perspective montrant plus particulièrement un dispositif de lecture de code d'identification appartenant au système d'analyse.
Figure 14 est une vue en perspective montrant plus particulièrement le chariot de convoyage en regard de la zone de chargement.
Figures 15 et 16 sont des vues en perspective montrant plus particulièrement le chariot de convoyage en regard d'une zone de déchargement appartenant au système d'analyse.
Figure 17 est une vue en perspective d'un rotor de stockage appartenant au système d'analyse.
Figures 18 et 19 sont des vues en perspective montrant plus particulièrement un dispositif de transfert appartenant au système d'analyse.

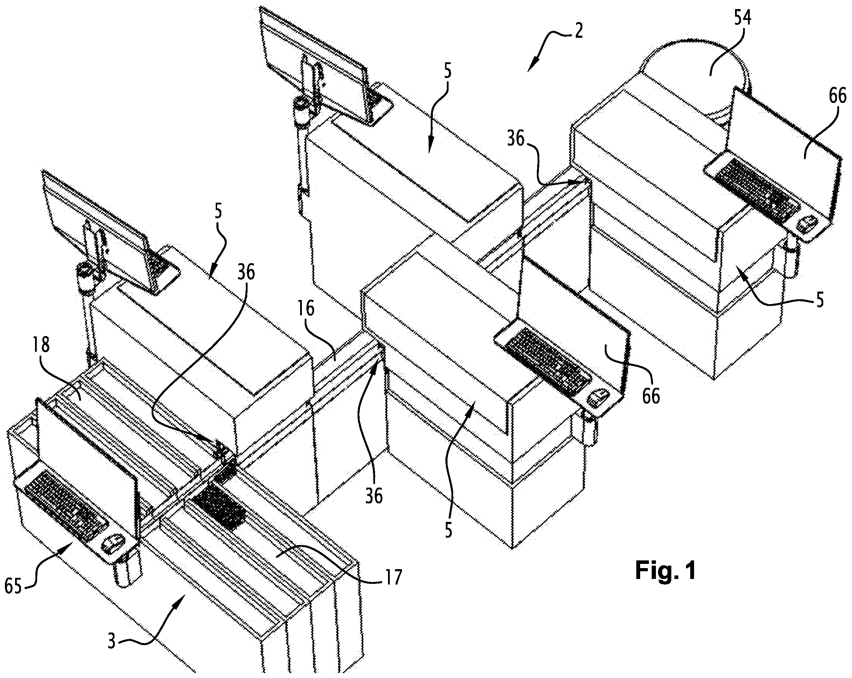

La figure 1 représente un système d'analyse automatique 2 pour diagnostic in vitro comprenant un système de convoyage 3 configuré pour convoyer des supports de récipients 4, et une pluralité de postes d'analyse et/ou de mesure 5 disposés le long du système de convoyage 3. Chaque poste d'analyse et/ou de mesure 5 peut par exemple comporter un ou plusieurs modules choisis notamment parmi un module de lecture spectrophotométrique, un module de lecture par fluorescence, un module de lecture par luminescence, et un module de mesure de coagulation.

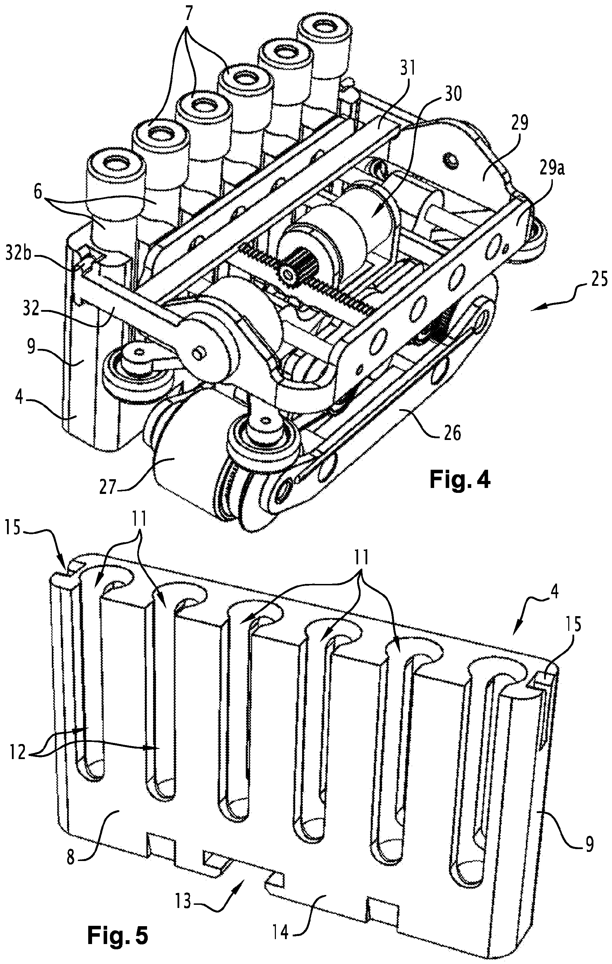

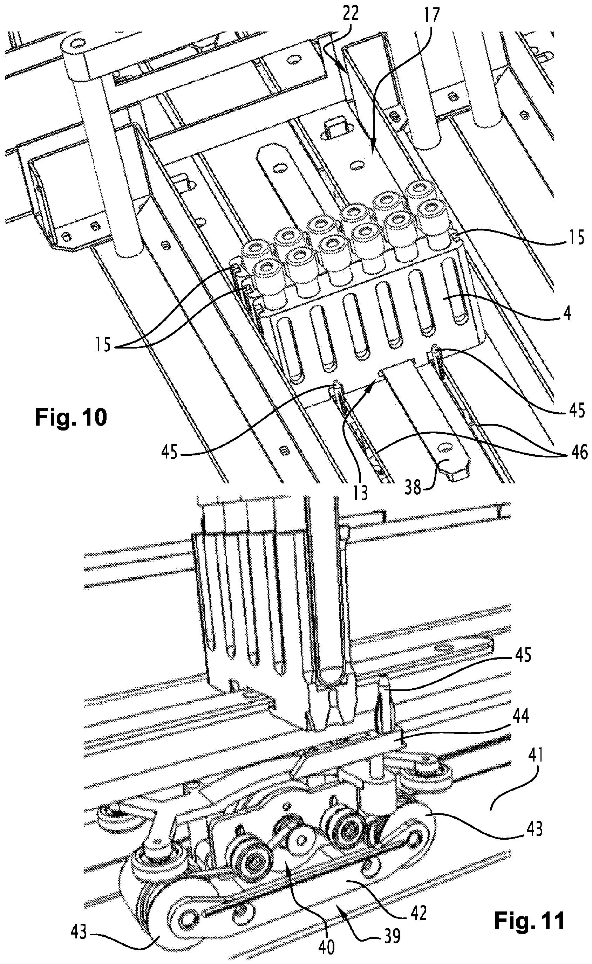

Comme montré plus particulièrement sur les figures 5 et 10, le système de convoyage 3 comprend une pluralité de supports de récipients 4, également appelés casiers, cassettes ou portoir, destinés chacun à supporter une pluralité de récipients 6 équipés d'éléments d'obturation 7 et contenants des échantillons de liquide biologique à analyser, tels que des échantillons de sang, de plasma ou encore de sérum sanguin. Avantageusement, les récipients 6 sont des tubes d'échantillons.

Chaque support de récipients 4 présente une forme générale parallélépipédique, et s'entend selon direction d'extension. Chaque support de récipients 4 comporte plus particulièrement deux faces longitudinales 8 opposées l'une à l'autre, et deux faces transversales 9 opposées l'une à l'autre.

Chaque support de récipients 4 comporte une pluralité de logements de réception 11, de préférence cylindriques, alignés selon la direction d'extension dudit support de récipients. Les logements de réception 11 sont avantageusement ouverts vers le haut afin de permettre une introduction et un retrait aisés des récipients 6 dans et hors des logements de réception. Selon le mode de réalisation représenté sur les figures, chaque support de récipients 4 comporte six logements de réception 11, et est donc configuré pour recevoir six récipients 6. Cependant, chaque support de récipients 4 pourrait comporter moins ou plus de six logements de réception 11.

Chaque support de récipients 4 comprend une pluralité de fenêtres de lecture 12 permettant une lecture optique de codes d'identification portés par les récipients 6 reçus sur ledit support de récipients.

Chaque support de récipients comprend de plus une encoche de guidage 13 ménagée son embase 14, et deux encoches d'accrochage 15 ménagées respectivement sur ses deux faces transversales 9. Chaque encoche d'accrochage 15 est configurée pour s'étendre sensiblement verticalement en conditions d'utilisation, et s'étend avantageusement parallèlement à la direction d'extension des logements de réception 11 respectifs.

Le système de convoyage 3 comprend en outre une unité de convoyage 16 configurée pour convoyer des supports de récipients 4 vers les postes d'analyse et/ou de mesure 5, au moins une zone de chargement 17 configurée pour stocker et charger des supports de récipients 4 dans l'unité de convoyage 16, et au moins une zone de déchargement 18 configurée pour stocker et recevoir des supports de récipients 4 déchargés de l'unité de convoyage 16.

L'unité de convoyage 16 comprend plus particulièrement un premier et un deuxième éléments de guidage de support 19, 21 définissant respectivement un premier et un deuxième chemins de guidage rectilignes et parallèles. Les premier et deuxième éléments de guidage de support 19, 21 sont par exemple chacun formé par une glissière de guidage. Les premier et deuxième éléments de guidage de support 19, 21 sont chacun configurés pour recevoir au moins l'embase 14 d'un support de récipients 4. Le premier élément de guidage de support 19 est plus particulièrement configuré pour guider en translation un support de récipients 4 le long du premier chemin de guidage, tandis que le deuxième élément de guidage de support 21 est plus particulièrement configuré pour guider en translation un support de récipients 4 le long du deuxième chemin de guidage.

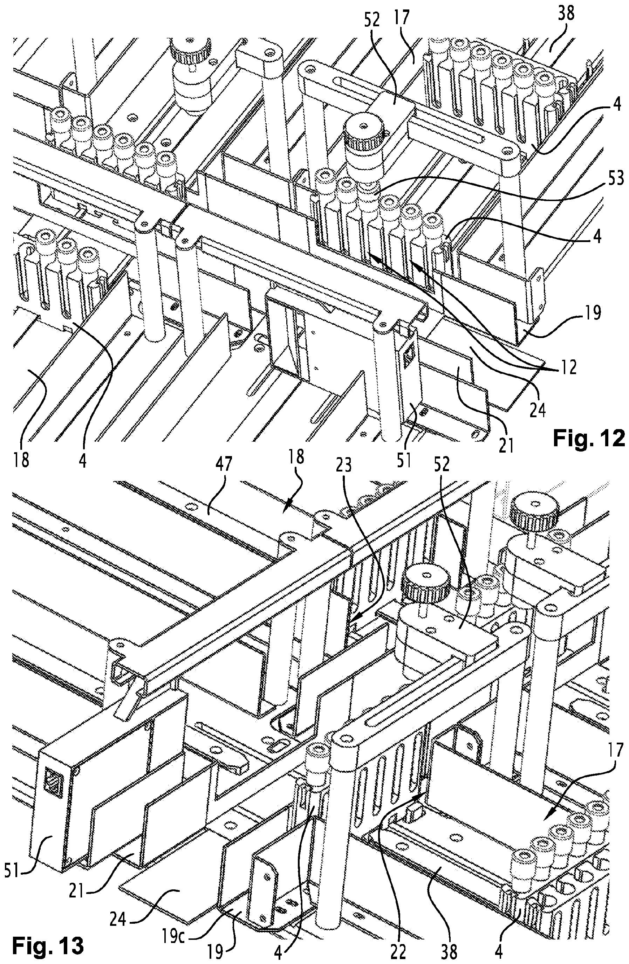

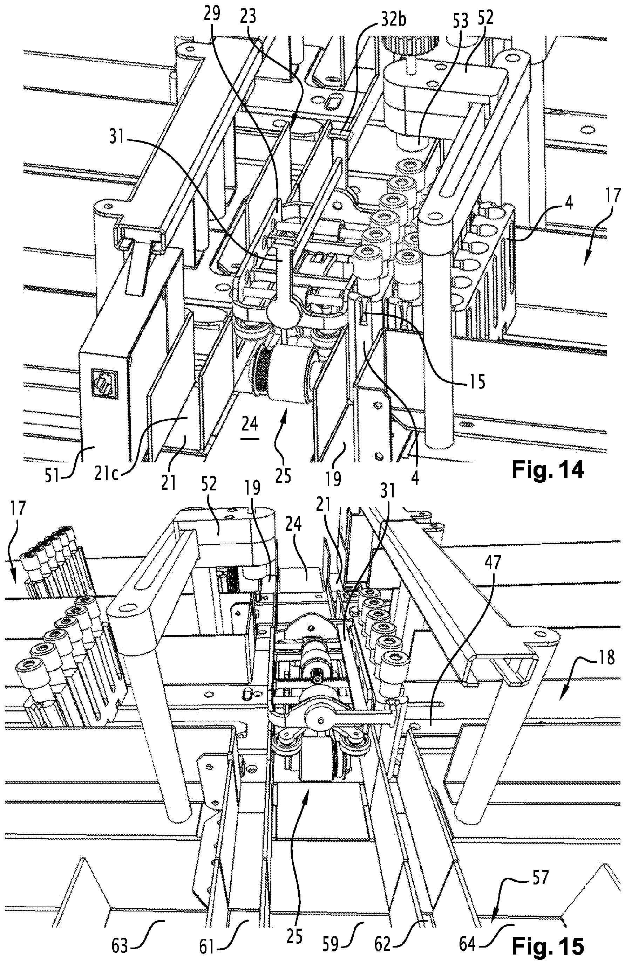

Comme montré plus particulièrement sur les figures 10 et 13, le premier élément de guidage de support 19 comporte une ouverture de chargement 22 disposée en regard de la zone de chargement 17 et destinée au passage d'un support de récipients 4, et, comme montré plus particulièrement sur les figures 13 et 14, le deuxième élément de guidage de support 21 comporte une ouverture de déchargement 23 disposée en regard de la zone de déchargement 18 et destinée au passage d'un support de récipients 4.

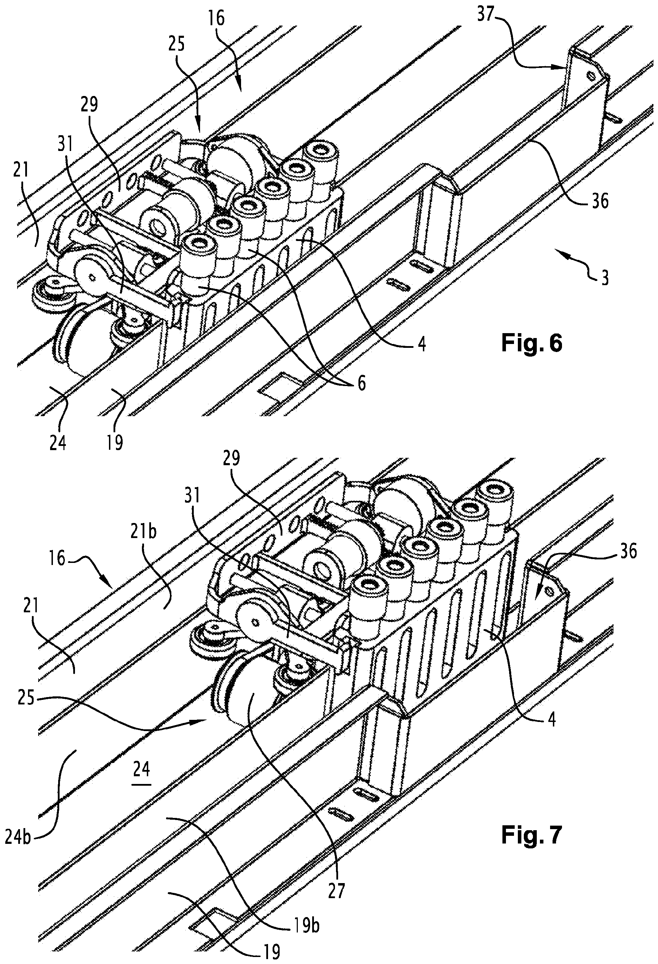

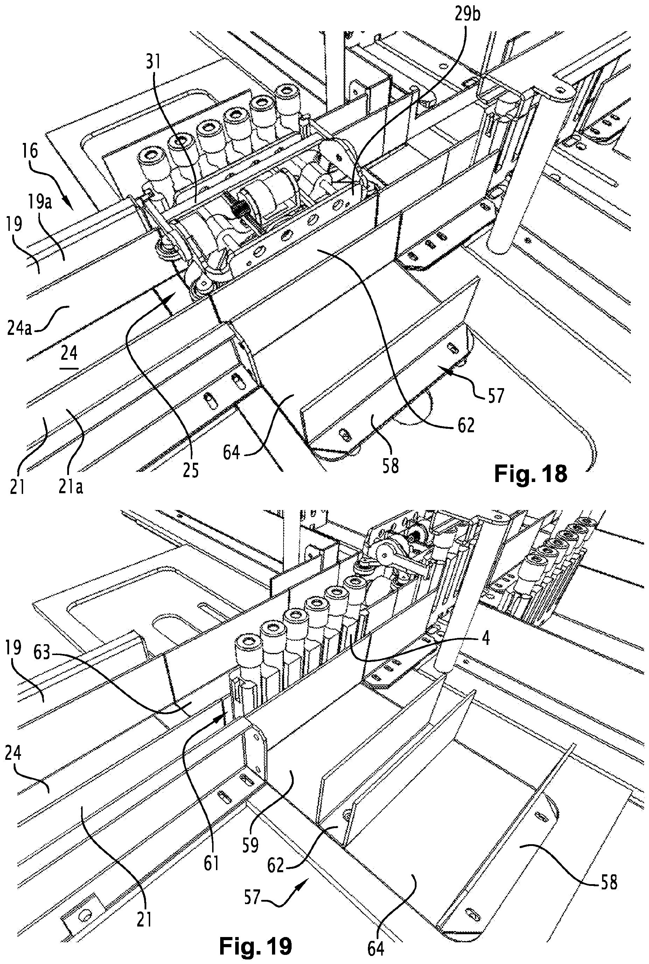

Chacun des premier et deuxième éléments de guidage de support 19, 21 présente avantageusement une largeur correspondant sensiblement à la largeur des supports de récipients 4. Ainsi, chacun des premier et deuxième éléments de guidage de support 19, 21 comporte une première surface de guidage latérale 19a, 21a configurée pour coopérer avec une première face longitudinale 8 d'un support de récipients 4, et une deuxième surface de guidage latérale 19b, 21b configurée pour coopérer avec une deuxième face longitudinale 8 d'un support de récipients 4 (voir notamment les figures 7 et 18).

Chacun des premier et deuxième éléments de guidage de support 19, 21 comporte également une surface de fond 19c, 21c agencée pour coopérer avec la surface inférieure de l'embase 14 d'un support de récipients 4 lors des déplacements de ce dernier le long du chemin de guidage respectif.

L'unité de convoyage 16 comprend également un élément de guidage de chariot 24 disposé entre les premier et deuxième éléments de guidage de support 19, 21, et définissant un chemin de convoyage qui est rectiligne et parallèle aux premier et deuxième chemins de guidage. L'élément de guidage de chariot 24 est par exemple formé par une glissière de guidage. L'élément de guidage de chariot 24 comporte deux surface de guidage latérales 24a, 24b opposée l'un par rapport à l'autre, et une surface de fond 24c (voir notamment les figures 7 et 18).

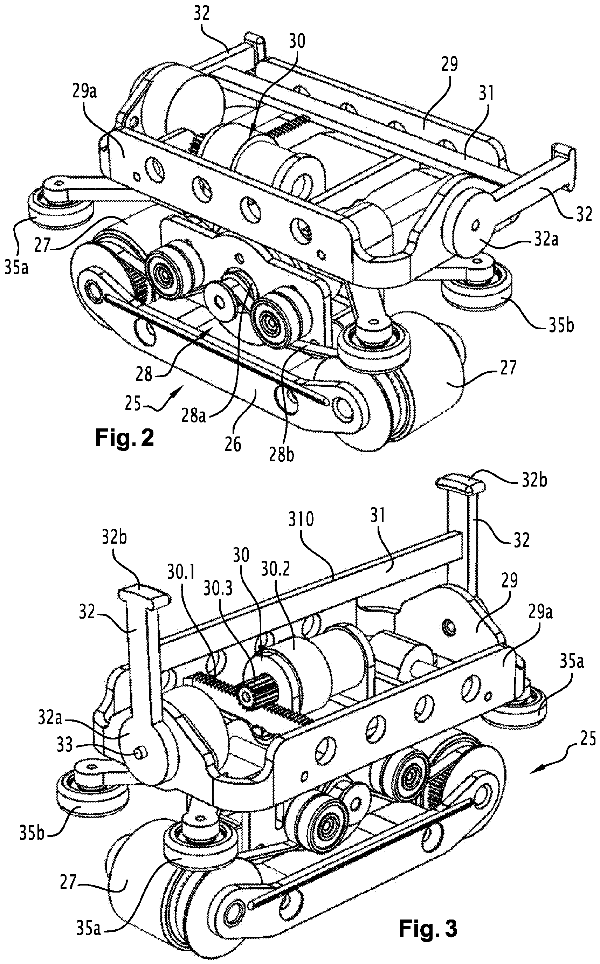

L'unité de convoyage 16 comprend de plus un chariot de convoyage autopropulsé 25 déplaçable le long du chemin de convoyage. Comme montré plus particulièrement sur les figures 2 et 3, le chariot de convoyage autopropulsé 25 comporte un corps de chariot 26, et deux roues motrices 27 montées rotatives sur le corps de chariot 26 et destinée à rouler sur la surface de fond 24c de l'élément de guidage de chariot. Chaque roue motrice 27 présente un axe de rotation s'étendant perpendiculairement aux premier et deuxième chemins de guidage.

Le chariot de convoyage autopropulsé 25 comporte un mécanisme d'entraînement en rotation 28 configuré pour entraîner en rotation les deux roues motrices 27. Le mécanisme d'entraînement en rotation 28 comprend par exemple un moteur d'entraînement 28a couplé en rotation aux roues motrices 27 par une courroie d'entraînement 28b.

Chaque roue motrice 27 peut être entraînée en rotation dans un premier sens de rotation et dans un deuxième sens de rotation opposé au premier sens de rotation. Ainsi, le chariot de convoyage autopropulsé 25 est déplaçable le long du chemin de convoyage dans un premier sens de déplacement et dans un deuxième sens de déplacement opposé au premier sens de déplacement.

Le chariot de convoyage autopropulsé 25 comporte en outre un élément de support 29, par exemple sous la forme d'un cadre de support s'étendant horizontalement, monté mobile en translation par rapport au corps de chariot 26 selon une direction de déplacement horizontale et perpendiculaire au chemin de convoyage. La direction de déplacement de l'élément de support 29 est avantageusement perpendiculaire à la direction d'extension du corps de chariot 26.

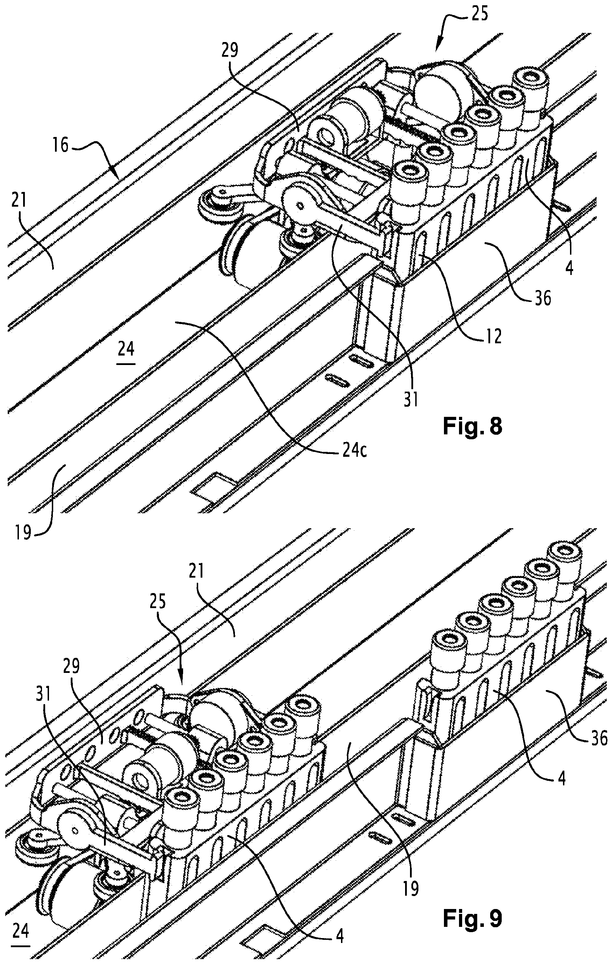

L'élément de support 29 peut occuper plus particulièrement une position de convoyage (voir la figure 6) dans laquelle l'élément de support 29 s'étend intégralement ou sensiblement intégralement au dessus du chemin de convoyage, une première position de dégagement (voir la figure 8) dans laquelle l'élément de support 29 s'étend en partie au dessus du premier chemin de guidage, et une deuxième position de dégagement dans laquelle l'élément de support 29 s'étend en partie au dessus du deuxième chemin de guidage.

L'élément de support 29 comprend en outre une première surface de poussée 29a configurée pour exercer un effort de poussée contre un support de récipients 4 reçu dans le premier élément de guidage de support 19 lorsque l'élément de support 29 est déplacé dans la première position de dégagement, et une deuxième surface de poussée 29b, opposée à la première surface de poussée 29a, configurée pour exercer un effort de poussée contre un support de récipients 4 reçu dans le deuxième élément de guidage de support 21 lorsque l'élément de support 29 est déplacé dans la deuxième position de dégagement. Chacune des première et deuxième surfaces de poussée 29a, 29b est plus particulièrement configurée pour prendre appui contre une face longitudinale 8 d'un support de récipients 4.

Le chariot de convoyage autopropulsé 25 comporte également un mécanisme d'entraînement en translation 30 configuré pour déplacer en translation l'élément de support 29 par rapport au corps de chariot 26, et plus particulièrement pour déplacer l'élément de support 29 entre les position de convoyage et les première et deuxième positions de dégagement. Le mécanisme d'entraînement en translation 30 peut par exemple comporter une crémaillère 30.1 prévue sur l'élément de support 29 et un moteur d'entraînement 30.2 prévu sur le corps de chariot 26 et solidaire en rotation d'une roue dentée 30.3 configurée pour coopérer avec la crémaillère 30.1. Le mécanisme d'entraînement en translation 30 pourrait comporter d'autres types d'actionneurs connus de l'homme du métier, tel qu'un vérin comprenant une première portion reliée à l'élément de support 29 et une deuxième portion reliée au corps de chariot 26.

Il convient d'être noté que le chariot de convoyage autopropulsé 25 et les premier et deuxième éléments de guidage de support 19, 21 sont agencés pour maintenir les supports de récipients 4 sensiblement verticaux lors de leurs déplacements le long du chemin de guidage respectif.

Comme montré plus particulièrement sur les figures 2 et 3, le chariot de convoyage autopropulsé 25 comporte de plus un élément d'entraînement 31, par exemple sous la forme d'une fourche d'entraînement, monté pivotant sur l'élément de support 29 autour d'un axe de pivotement s'étendant sensiblement parallèlement au chemin de convoyage et à la direction d'extension du corps de chariot 26. L'élément d'entraînement 31 comprend plus particulièrement deux branches d'entraînement 32 espacées l'une de l'autre d'une distance correspondant sensiblement à la longueur des supports de récipients 4.

Chaque branche d'entraînement 32 comporte une portion de montage 32a montée pivotante sur l'élément de support 29, et un doigt d'accrochage 32b configuré pour être inséré dans une encoche d'accrochage 15 respective ménagée sur une face transversale 9 d'un support de récipients 4. L'élément d'entraînement 31 comporte avantageusement une partie de liaison 310 reliant les deux branches d'accrochage et s'étendant parallèlement à l'axe de pivotement de l'élément d'entraînement 31.

L'élément d'entraînement 31 peut occuper plus particulièrement une première position d'entraînement (voir la figure 4) dans laquelle les doigts d'accrochage 32b peuvent être insérés dans les encoches d'accrochage 15 ménagées sur un support de récipient 4 reçu dans le premier élément de guidage de support 19 et l'élément d'entraînement 29 peut transmettre un mouvement d'entraînement au support de récipients 4 reçu sur dans le premier élément de guidage de support 19, une deuxième position d'entraînement (voir la figue 15) dans laquelle les doigts d'accrochage 32b peuvent être insérés dans les encoches d'accrochage 15 ménagées sur un support de récipient 4 reçu dans le deuxième élément de guidage de support 21 et l'élément d'entraînement 31 peut transmettre un mouvement d'entraînement au support de récipients 4 reçu dans le deuxième élément de guidage de support 21, et une position de libération (voir les figures 3 et 14) dans laquelle l'élément d'entraînement 31 peut libérer lesdits supports de récipients 4. Avantageusement, l'élément d'entraînement 31 s'étend sensiblement horizontalement lorsqu'il se trouve dans les première et deuxième positions d'entraînement. L'élément d'entraînement 31 peut par exemple s'étendre sensiblement verticalement lorsqu'il se trouve dans la position de libération.

Ainsi, le chariot de convoyage autopropulsé 25 est plus particulièrement configuré pour déplacer en translation un support de récipients 4 reçu dans le premier élément de guidage de support 19 le long du premier chemin de guidage lorsque le chariot de convoyage autopropulsé 25 se déplace le long du chemin de convoyage et lorsque l'élément d'entraînement 31 est dans la première position d'entraînement, et pour déplacer en translation un support de récipients 4 reçu dans le deuxième élément de guidage de support 21 le long du deuxième chemin de guidage lorsque le chariot de convoyage autopropulsé 25 se déplace le long du chemin de convoyage et lorsque l'élément d'entraînement 31 est dans la deuxième position d'entraînement.

Le chariot de convoyage autopropulsé 25 comporte un dispositif d'actionnement 33 configuré pour faire pivoter l'élément d'entraînement 31 autour de son axe de pivotement et entre les première et deuxième positions d'entraînement et la position de libération. Le dispositif d'actionnement 33 peut comporter différents types d'actionneurs, tels qu'un moteur couplé en rotation à l'élément d'entraînement 31.

Le chariot de convoyage autopropulsé 25 comporte également une batterie (non visible sur les figures) configurée pour alimenter électriquement le chariot de convoyage autopropulsé, et plus particulièrement le mécanisme d'entraînement en translation 30, le mécanisme d'entraînement en rotation 28 et le dispositif d'actionnement. Selon le mode de réalisation représenté sur les figures, la batterie est rechargeable, et peut être rechargée par exemple par contact ou par induction. A cet effet, le système de convoyage 3 comporte une zone de rechargement (non visible sur les figures) comportant un dispositif de rechargement électrique configuré pour recharger électriquement la batterie lorsque le chariot de convoyage autopropulsé 25 est situé dans la zone de rechargement.

Selon le mode de réalisation représenté sur les figures, le chariot de convoyage autopropulsé 25 comporte en outre une première paire de galets de guidage 35a configurés pour coopérer avec la première surface de guidage 24a, et une deuxième paire de galets de guidage 35b configurés pour coopérer avec la deuxième surface de guidage 24b. Chaque galet de guidage 35a, 35b présente avantageusement un axe de rotation sensiblement vertical.

Le système de convoyage 3 comprend également une pluralité de zones de prélèvement 36 disposées le long de l'unité de convoyage 16. Selon le mode de réalisation représenté sur les figures, le système de convoyage 3 comporte une pluralité de zones de prélèvement 36, deux dans le cas représenté sur les figures, disposées le long du premier chemin de guidage et à l'extérieur du premier chemin de guidage, et pluralité de zones de prélèvement 36, deux dans le cas représenté sur les figures, disposées le long du deuxième chemin de guidage et à l'extérieur du deuxième chemin de guidage.

Chaque zone de prélèvement 36 comporte un emplacement de prélèvement disposé à proximité d'un poste d'analyse et/ou de mesure 5 respectif, et agencé pour recevoir et stocker au moins temporairement un support de récipients 4. Ainsi, chacun des premier et deuxième éléments de guidage de support 19, 21 comporte une pluralité d'ouvertures de passage 37 chacune débouchant dans la zone de prélèvement 36 respective, et chacune destinée au passage d'un support de récipients 4 depuis le chemin de guidage respectif.

Comme montré plus particulièrement sur les figures 7 et 8, l'élément de support 29 et l'élément d'entraînement 31 du chariot de convoyage autopropulsé 25 sont configurés de telle sorte que, lorsque le chariot de convoyage autopropulsé 25 est disposé en regard d'une zone de prélèvement 36 disposée le long du premier chemin de guidage et l'élément d'entraînement 31 est dans la première position d'entraînement et est accouplé à un support de récipient 4 reçu dans le premier chemin de guidage, un déplacement de l'élément de support 29 de la position de convoyage à la première position de dégagement entraîne un déplacement du support de récipients 4 dans ladite zone de prélèvement 36 de manière à libérer le premier chemin de guidage. Dans une telle position de l'élément de support 29, l'élément d'entraînement 31 peut bien entendu être déplacé dans la position de libération afin de libérer le support de récipient 4 reçu dans la zone de prélèvement 36, et le chariot de convoyage 25 peut alors se déplacer le long du chemin de convoyage par exemple pour aller saisir un autre support de récipient 4.

L'élément de support 29 et l'élément d'entraînement 31 du chariot de convoyage autopropulsé 25 sont également configurés de telle sorte que, lorsque le chariot de convoyage autopropulsé 25 est disposé en regard d'une zone de prélèvement 36 disposée le long du premier chemin de guidage et recevant un support de récipient 4, l'élément d'entraînement 31 peut dans un premier temps saisir ledit support de récipient 4 par des déplacements successifs de l'élément de support 29 dans la première position de dégagement et de l'élément d'entraînement 31 dans la première position d'entraînement, et dans un second temps déplacer le support de récipients 4 saisi dans le premier chemin de guidage par un déplacement de l'élément de support 29 dans la position de convoyage.

De façon similaire, l'élément de support 29 et l'élément d'entraînement 31 du chariot de convoyage autopropulsé 25 sont également configurés pour déplacer un support de récipient 4 reçu dans le deuxième chemin de guidage vers une zone de prélèvement 36 disposée le long du deuxième chemin de guidage, et pour déplacer un support de récipient 4 reçu dans une zone de prélèvement 36 disposée le long du deuxième chemin de guidage vers le deuxième chemin de guidage.

Avantageusement, chaque poste d'analyse et/ou de mesure 5 comporte un dispositif de prélèvement (non représenté sur les figures) disposé de manière adjacente à la zone de prélèvement 36 respective, et configuré pour prélever des échantillons dans les récipients 6 supportés par un support de récipients 4 reçu dans la zone de prélèvement 36 respective. Avantageusement, chaque dispositif de prélèvement comporte une tête de prélèvement équipée d'une aiguille de prélèvement, des premiers moyens de déplacement agencés pour déplacer en translation la tête de prélèvement respective suivant une direction sensiblement horizontale et sensiblement parallèle au chemin de convoyage, et des seconds moyens de déplacement agencés pour déplacer en translation la tête de prélèvement respective suivant une direction sensiblement verticale.

Avantageusement, chaque poste d'analyse et/ou de mesure 5 comporte également des moyens de détection agencés pour détecter la réception d'un support de récipients 4 dans la une zone de prélèvement 36 respective.

Comme montré plus particulièrement sur la figure 10, la zone de chargement 17 comporte un rail de guidage 38 s'étendant perpendiculairement au chemin de convoyage. Le rail de guidage 38 est configuré pour coopérer avec l'encoche de guidage 13 de chaque support de récipients 4 stocké dans la zone de chargement 17. Le rail de guidage 38 est plus précisément agencé pour guider en translation chaque support de récipients 4 stocké dans la zone de chargement 17 selon une direction de chargement lors de leurs déplacements dans la zone de chargement 17 et leur chargement dans l'unité de convoyage 16, et plus particulièrement dans le premier élément de guidage de support 19. Avantageusement, la direction de chargement s'étend sensiblement perpendiculairement à la direction d'extension de chaque support de récipients 4 stocké dans la zone de chargement.

La zone de chargement 17 comporte également un dispositif de chargement (voir notamment la figure 11) agencé d'une part pour déplacer les supports de récipients 4 stockés dans la zone de chargement 17 en direction de l'unité de convoyage 16 et selon la direction de chargement, et d'autre part pour charger chaque support de récipients 4, stocké dans la zone de chargement, dans le premier chemin de guidage défini par le premier élément de guidage de support 19.

Selon le mode de réalisation représenté sur les figures, le dispositif de chargement comporte un chariot de chargement autopropulsé 39 déplaçable le long d'un chemin de chargement définit par un élément de guidage de chariot 41, par exemple sous la forme d'une glissière de guidage, disposé sous le rail de guidage 38. Le chariot de chargement autopropulsé 39 comporte un corps de chariot 42, et deux roues motrices 43 montées rotatives sur le corps de chariot 42 et destinées à rouler sur la surface de fond de l'élément de guidage de chariot 41. Chaque roue motrice 43 présente un axe de rotation s'étendant parallèlement aux premier et deuxième chemins de guidage. Le chariot de chargement autopropulsé 39 comporte un mécanisme d'entraînement en rotation 40 configuré pour entraîner en rotation les deux roues motrices 43. Le mécanisme d'entraînement en rotation 40 comprend par exemple un moteur d'entraînement couplé en rotation aux roues motrices 43 par l'intermédiaire d'une courroie d'entraînement.

Chaque roue motrice 43 peut être entraînée en rotation dans un premier sens de rotation et dans un deuxième sens de rotation opposé au premier sens de rotation. Ainsi, le chariot de chargement autopropulsé 39 est déplaçable le long du chemin de chargement dans un premier sens de déplacement et dans un deuxième sens de déplacement opposé au premier sens de déplacement.

Le chariot de chargement autopropulsé 39 comporte de plus un élément d'entraînement 44, par exemple sous la forme d'une fourche d'entraînement, monté mobile en translation par rapport au corps de chariot 42 selon une direction de déplacement sensiblement verticale. L'élément d'entraînement 44 comprend plus particulièrement deux branches d'entraînement 45 (voir les figures 10 et 11) destinées à s'étendre à travers deux fentes 46 parallèles s'étendant de part et d'autre du rail de guidage 38 et à coopérer avec l'embase 14 d'un support de récipients 4 stocké dans la zone de chargement 17.

L'élément d'entraînement 44 est plus particulièrement monté mobile en translation entre une position d'entraînement dans laquelle les deux branches d'entraînement 45 font saillie des fentes 46 et sont configurées pour coopérer avec l'embase 14 d'un support de récipients 4 stocké dans la zone de chargement 17 et l'élément d'entraînement 44 est configuré pour transmettre un mouvement d'entraînement audit support de récipients 4, et une position de libération dans laquelle les deux branches d'entraînement 45 sont disposées en retrait des fentes 46 ou du moins en dessous de la surface inférieure dudit support de récipients 4.

Ainsi, le chariot de chargement autopropulsé 39 est configuré pour déplacer en translation chaque support de récipients 4 le long du chemin de chargement, et pour charger successivement chaque support de récipients 4 dans le premier chemin de guidage lorsque le chariot de chargement autopropulsé 39 se déplace le long du chemin de chargement et lorsque l'élément d'entraînement 44 est dans la position d'entraînement.

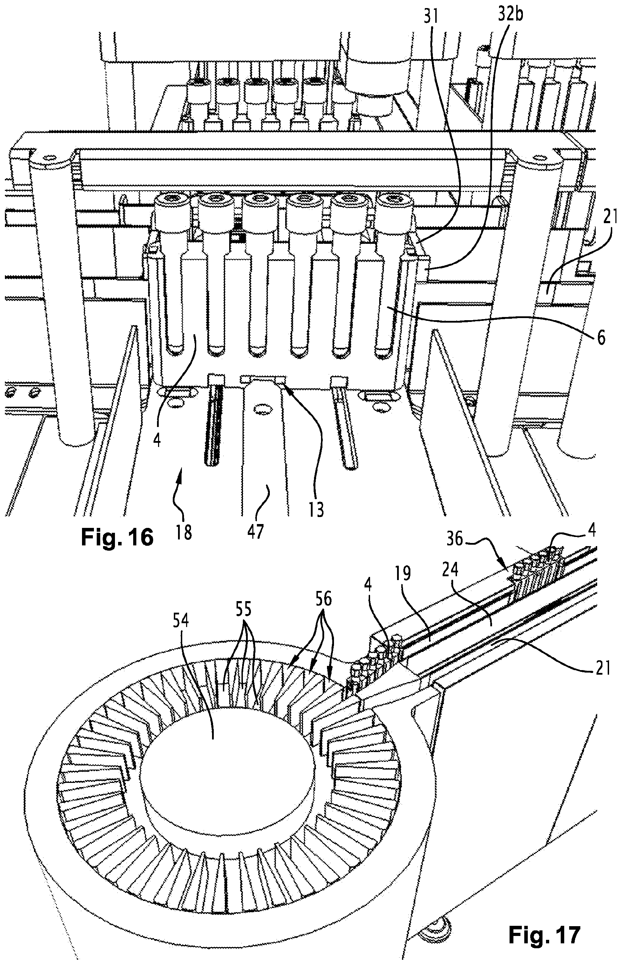

Comme montré plus particulièrement sur les figures 15 et 16, la zone de déchargement 18 comporte un rail de guidage 47 s'étendant perpendiculairement au chemin de convoyage. Le rail de guidage 47 est agencé pour guider en translation chaque support de récipients 4 déchargé dans la zone de déchargement 18 depuis le deuxième chemin de guidage selon une direction de déchargement. Le rail de guidage 47 est plus particulièrement configuré pour coopérer avec l'encoche de guidage 13 de chaque support de récipients 4 déchargé dans la zone de déchargement 18. Avantageusement, la direction de déchargement s'étend sensiblement perpendiculairement à la direction d'extension de chaque support de récipients 4 déchargé dans la zone de déchargement 18.

Selon le mode de réalisation représenté sur les figures, le chariot de convoyage autopropulsé 25 est configuré pour déplacer un support de récipients 4, disposé dans le deuxième chemin de support, dans la zone de déchargement 18. En particulier, l'élément de support 29 et l'élément d'entraînement 31 sont configurés de telle sorte que, lorsque le chariot de convoyage autopropulsé 25 est disposé en regard de la zone de déchargement 18 et l'élément d'entraînement 31 est dans la position d'entraînement et est accouplé à un support de récipients 4 reçu dans le deuxième chemin de guidage, un déplacement de l'élément de support 29 de la position de convoyage à la deuxième position de dégagement entraîne un déplacement du support de récipients 4 dans la zone de déchargement 18.

Avantageusement, le système de convoyage 3 comporte une pluralité de repères de positionnement (non visibles sur les figures) disposés sur le chemin de convoyage. Le système de convoyage 3 comporte par exemple un repère de positionnement en regard de chaque zone de prélèvement 36, un repère de positionnement en regard de la zone de chargement 17 et un repère de positionnement en regard de la zone de déchargement 18. Selon un tel mode de réalisation de l'invention, le chariot de convoyage autopropulsé 25 comporte d'une part des moyens de détection, tels qu'un lecteur optique, un détecteur RFID ou un détecteur inductif, agencés pour détecter les repères de positionnement disposés sur le chemin de convoyage lors des déplacements du chariot de convoyage autopropulsé 25 le long du chemin de convoyage, et d'autre part des moyens de commande, tels qu'un circuit intégré ou un microprocesseur, agencés pour commander l'immobilisation du chariot de convoyage autopropulsé 25 lorsque les moyens de détection détectent le repère de positionnement associé à la zone du système de convoyage 3 que le chariot de convoyage autopropulsé 25 doit atteindre. Chaque repère de positionnement peut être formé par exemple par une barrière optique, un code-barres, un code QR ou encore une étiquette RFID.

Comme monté sur les figures 12 et 13, le système de convoyage 3 comporte également un dispositif de lecture de code d'identification 51 configuré pour lire optiquement des codes d'identification portés par les récipients 6 supportés par un support de récipients 4 disposé dans le premier chemin de guidage et en regard de la zone de chargement 17. Les informations enregistrées dans chaque code d'identification peuvent par exemple être le numéro de référence de l'échantillon respectif. Avantageusement, les codes d'identification portés par les récipients 6 sont formés par des codes-barres ou des codes QR disposés sur la surface extérieure des récipients. Avantageusement, le dispositif de lecture de code d'identification 51 est monté mobile en translation selon une direction de déplacement sensiblement parallèle au chemin de convoyage afin de pouvoir aisément lire optiquement les codes d'identification portés par les différents récipients 6 d'un même support de récipients 4.

Il convient d'être noté que les différentes destinations de convoyage d'un support de récipients 4 sont avantageusement déterminées en fonction des codes d'identification portés par les différents récipients 4 portés par ledit support de récipients 4.

Comme monté sur les figures 12 à 14, le système de convoyage 3 comporte en outre un module d'entraînement en rotation 52 configuré pour entraîner en rotation des récipients 6 supportés par un support de récipients 4 disposé dans le premier chemin de guidage et en regard de la zone de chargement 17, de manière à permettre la lecture optique des codes d'identification portés par lesdits récipients 6 par le dispositif de lecture de code d'identification 51 à travers les fenêtres de lecture 12 respectives. Avantageusement, le module d'entraînement en rotation 52 comporte un organe d'actionnement 53 monté mobile en rotation autour d'un axe vertical, et agencé pour entraîner en rotation un récipient 6 autour de son axe d'extension. Selon le mode de réalisation représenté sur les figures, le module d'entraînement en rotation 53 comprend des premiers moyens de déplacement agencés pour déplacer en translation l'organe d'actionnement 53 suivant une première direction de déplacement sensiblement horizontale et parallèle au chemin de convoyage, et des seconds moyens de déplacement agencés pour déplacer en translation l'organe d'actionnement 53 suivant une seconde direction de déplacement sensiblement verticale.

Comme monté sur les figures 1 et 17, le système de convoyage 3 comporte également un rotor de stockage 54, d'axe de rotation sensiblement vertical, disposé à une extrémité du chemin de convoyage. Le rotor de stockage 54 comporte une pluralité de logements de stockage 55 décalés angulairement et configurés chacun pour stocker un support de récipients 4 provenant des premier et deuxième chemins de guidage. Chaque logement de stockage 55 s'étend radialement, et comporte plus particulièrement une ouverture d'introduction radiale 56 apte à être disposée en regard des premier et deuxième chemins de guidage en fonction de la position angulaire du rotor de stockage 54.

Le système de convoyage 3 comporte avantageusement des moyens d'entraînement en rotation associés au rotor de stockage 54, et agencés pour entraîner en rotation le rotor de stockage 54 autour de son axe de rotation dans un premier sens et dans un deuxième sens opposé au premier sens. Ainsi, le rotor de stockage 54 est également configuré pour transférer un support d'échantillon 4 du premier chemin de guidage au deuxième chemin de guidage, et inversement.

Comme monté plus particulièrement sur les figures 15, 18 et 19, le système de convoyage 3 comporte un dispositif de transfert 57 disposé le long de l'unité de convoyage 16 et mobile en translation selon une direction de déplacement perpendiculairement au chemin de convoyage. Le dispositif de transfert 57 est configuré pour transférer un support de récipients du premier chemin de guidage au deuxième chemin de guidage, et inversement.

Le dispositif de transfert 57 comporte plus particulièrement une plaque de transfert 58 comportant une partie de convoyage principale 59, une première et une deuxième parties de transfert 61, 62 disposées de part et d'autre de la partie de convoyage principale 59, et une première et une deuxième parties de convoyage secondaires 63, 64 disposées de part et d'autre des première et deuxième parties de transfert 61, 62.

Le dispositif de transfert 57 est déplaçable entre une position de convoyage (voir la figure 18) dans laquelle les première et deuxième parties de transfert 61, 62 définissent en partie respectivement les premier et deuxième chemins de guidage et la partie de convoyage principale 59 définit en partie le chemin de convoyage, une première position de transfert (voir la figure 19) dans laquelle la première partie de transfert 61 et la première partie de convoyage secondaire 63 définissent en partie respectivement le deuxième chemin de guidage et le chemin de convoyage, et une deuxième position de transfert dans laquelle la deuxième partie de transfert 62 et la deuxième partie de convoyage secondaire 64 définissent en partie respectivement le premier chemin de guidage et le chemin de convoyage.

Comme montré sur la figure 1, le système de convoyage 3 comporte une unité de commande 65 configurée pour communiquer sans fil, par exemple par wifi ou Bluetooth, avec le chariot de convoyage autopropulsé 25 et avec le chariot de chargement autopropulsé 39. L'unité de commande 65 peut être un ordinateur, par exemple de type PC. Avantageusement, l'unité de commande 65 est également configurée pour communiquer avec les différents postes d'analyse et/ou de mesure 5.

De façon avantageuse, les moyens de commande appartenant au chariot de convoyage autopropulsé 25 sont configurés pour recevoir des signaux de commande provenant de l'unité de commande 65, et pour transmettre des signaux d'entraînement notamment au mécanisme d'entraînement en translation, au mécanisme d'entraînement en rotation 28 et au dispositif d'actionnement, en réponse aux signaux de commande reçus.

Un exemple de procédé d'analyse d'échantillons pouvant être réalisé à l'aide du système d'analyse automatique 2 décrit précédemment va maintenant être décrit. Un tel procédé de traitement d'échantillons comprend notamment les étapes suivantes consistant à :
a) charger manuellement une pluralité de supports de récipient 4 dans la zone de chargement 17,
b) commander un déplacement du chariot de chargement autopropulsé 39 sensiblement à la verticale d'un support de récipient 4, et commander un déplacement en translation de l'élément d'entraînement 44 du chariot de chargement autopropulsé 39 dans la position d'entraînement (voir la figure 10) ;
c) déplacer le chariot de chargement autopropulsé 39 en direction de l'unité de convoyage 16 de manière à charger automatiquement un support de récipient 4 dans le premier chemin de guidage (voir la figure 12) ;
d) lire optiquement, à l'aide du dispositif de lecture de code d'identification 51, les codes d'identification portés par les différents récipients 6 supportés par le support de récipient 4 chargé dans le premier chemin de guidage et situé en regard de la zone de chargement 17 ;
e) optionnellement, entraîner en rotation un ou plusieurs des récipients 6 portés par le support de récipients 4 de manière à permettre la lecture optique de leurs codes d'identification par le dispositif de lecture de code d'identification 51 ;
f) déterminer les destinations de convoyage du support de récipients 4 chargé dans le premier chemin de guidage et situé en regard de la zone de chargement 17 en fonction des codes d'identification portés par les différents récipients 6 supportés par ledit support de récipients 4, et affecter ledit support de récipients 4 à un poste d'analyse et/ou de mesure 5 ;
g) commander un déplacement du chariot de convoyage autopropulsé 25 en regard de la zone de chargement 17 (voir la figure 14), et commander un pivotement de l'élément d'entraînement 31 du chariot de convoyage autopropulsé 25 dans la position d'entraînement de manière à saisir le support de récipients 4 ;
h) commander un déplacement du chariot de convoyage autopropulsé 25 en regard d'une zone de prélèvement 36 associée au poste d'analyse et/ou de mesure 5 auquel est affecté ledit support de récipients 4 (voir la figure 7), et commander une translation de l'élément de support 29 du chariot de convoyage autopropulsé 25 dans la première ou deuxième position de dégagement (en fonction du poste d'analyse et/ou de mesure 5 auquel est affecté le support de récipients 4) de manière à déplacer le support de récipient dans la zone de prélèvement 36 (voir la figure 8) ;
i) commander un pivotement de l'élément d'entraînement 31 du chariot de convoyage autopropulsé 25 dans la position de libération de manière à libérer le support de récipient 4 ;
j) prélever un échantillon dans un ou plusieurs récipients 6 supportés par ledit support de récipient 4, à l'aide du dispositif de prélèvement appartenant au poste d'analyse et/ou de mesure 5 associé à la zone de prélèvement 36, et traiter le ou les échantillon(s) prélevé(s) à l'aide du poste d'analyse et/ou de mesure ; pendant ces étapes de prélèvement et de traitement, le chariot de convoyage autopropulsé 25 peut être commandé pour déplacer un ou plusieurs autres supports de récipients 5 en temps masqué (voir la figure 9) ;
k) commander un déplacement du chariot de convoyage autopropulsé 25 en regard de la zone de prélèvement 36, commander une translation de l'élément de support 29 du chariot de convoyage autopropulsé 25 dans la première ou deuxième position de dégagement (en fonction du poste d'analyse et/ou de mesure 5 auquel est affecté le support de récipients 4), et commander un pivotement de l'élément d'entraînement 31 du chariot de convoyage autopropulsé 25 dans la position d'entraînement de manière à saisir le support de récipient 4 reçu dans la zone de prélèvement 36 ;
l) commander une translation de l'élément de support 29 du chariot de convoyage autopropulsé 25 dans la position de convoyage de manière à déplacer le support de récipient 4 dans le premier ou le deuxième chemins de guidage (en fonction du poste d'analyse et/ou de mesure 5 auquel est affecté le support de récipients 4) ;
m) commander un déplacement du chariot de convoyage autopropulsé 25 en regard de la zone de déchargement 18 (voir la figure 15), et commander une translation de l'élément de support 29 du chariot de convoyage autopropulsé 25 dans la deuxième position de dégagement de manière à décharger le support de récipient 4 dans la zone de déchargement 18 (voir la figure 16) ;
n) commander un pivotement de l'élément d'entraînement 31 du chariot de convoyage autopropulsé 25 dans la position de libération de manière à libérer le support de récipient 4.

Il convient d'être noté que les étapes a) à f) peuvent être réalisées pour un support de récipients 4 pendant que le chariot de convoyage autopropulsé 25 déplace un autre support de récipients 4. Ainsi, les étapes a) à f) peuvent être réalisées en temps masqué.

Afin d'augmenter la cadence du système de convoyage 3, ce dernier peut comprendre avantageusement plusieurs zones de chargements 17 disposées de manière adjacentes, et plusieurs zone de déchargements 18 disposées de manière adjacentes.

Un tel procédé d'analyse pourrait en outre comprend une étape réalisée entre les étapes h et h) et consistant à transférer le support de récipients 4 du premier chemin de guidage au deuxième chemin de guidage à l'aide du dispositif de transfert 57 si le poste d'analyse et/ou de mesure 5 auquel est affecté le support de récipients 4 est disposé le long du deuxième chemin de guidage.

Il convient en outre d'être noté le système d'analyse selon la présente invention est destiné à fluidifier les flux de traitement des échantillons dans un laboratoire d'analyse, afin d'en augmenter la productivité et la qualité (réduction de la main d'œuvre et des erreurs). Ainsi, il va de soi que le système de convoyage selon l'invention est configuré pour communiquer avec l'unité de commande 65, qui gère les charges de travail des différents postes d'analyse et/ou de mesure 5 (telles que les tests à effectuer par échantillon), et les transmet au système de convoyage 3 et aux postes d'analyse et/ou de mesure 5 de telle sorte que les différents récipients 6 soient acheminés vers les postes d'analyse et/ou de mesure 5 selon les demandes de tests et les capacités de chaque poste d'analyse et/ou de mesure 5. L'unité de commande 65, qui gère les chariots de convoyage et de chargement, dispose donc d'une « intelligence », une sorte d'ERP (progiciel de gestion intégré) pour optimiser les convoyages des supports de récipients 4 en fonction des charges de travail des différents postes d'analyse et/ou de mesure 5.

Il convient en outre d'être noté que chaque poste d'analyse et/ou de mesure 5 peut comprendre une interface de communication et de visualisation, et une électronique embarquée (non représentée sur les figures). Chaque interface de communication et de visualisation comporte par exemple un écran tactile 66 relié à un ordinateur de type PC. L'ordinateur de type PC est plus particulièrement agencé pour enregistrer des demandes d'analyses chargées manuellement par un opérateur à l'aide de l'écran tactile ou provenant de l'unité de commande 65, pour envoyer des requêtes d'analyse à l'électronique embarquée, pour récupérer des données mesurées, les traiter grâce à des algorithmes spécifiques, et mettre les résultats à disposition de l'opérateur ou les transmettre à l'unité de commande 65.

Selon une variante de réalisation de l'invention, au moins l'un des postes d'analyse et/ou de mesure 5 pourrait être remplacé par un dispositif d'analyse tel que décrit dans le document FR2998057. Selon un tel mode de réalisation, la zone de prélèvement 36 respective est remplacée par une zone de transfert permettant un transfert d'un support de récipient 4 du chemin de guidage respectif vers le dispositif d'analyse.

Comme il va de soi, l'invention ne se limite pas à la seule forme d'exécution de ce système de convoyage, décrite ci-dessus à titre d'exemple, elle en embrasse au contraire toutes les variantes de réalisation.

## Revendications

1. Système de convoyage (3) configuré pour convoyer des supports de récipients (4) destinés à supporter des récipients (6) contenant des échantillons de liquide biologique, le système de convoyage (3) comportant au moins :
- un élément de guidage de support (19) définissant un chemin de guidage, l'élément de guidage de support (19) étant configuré pour recevoir un support de récipients (4) et guider en translation ledit support de récipients (4) le long du chemin de guidage,
- un chariot de convoyage autopropulsé (25) déplaçable le long d'un chemin de convoyage s'étendant le long de l'élément de guidage de support (19), le chariot de convoyage autopropulsé (25) comprenant un élément d'entraînement (31) monté mobile entre au moins une position d'entraînement dans laquelle l'élément d'entraînement (31) est configuré pour transmettre un mouvement d'entraînement au support de récipients (4) reçu sur l'élément de guidage de support (19), et une position de libération dans laquelle l'élément d'entraînement (31) est configuré pour libérer le support de récipients (4), le chariot de convoyage autopropulsé (25) étant configuré pour déplacer en translation le support de récipients (4) le long du chemin de guidage lorsque l'élément d'entraînement (31) est dans la position d'entraînement et le chariot de convoyage autopropulsé (25) se déplace le long du chemin de convoyage.

2. Système de convoyage (3) selon la revendication 1, dans lequel l'élément d'entraînement (31) est monté pivotant autour d'un axe de pivotement.

3. Système de convoyage (3) selon la revendication 1 ou 2, dans lequel l'élément d'entraînement (31) comporte deux branches d'entraînement (32) espacées l'une de l'autre et configurées pour coopérer avec le support de récipients (4) lorsque l'élément d'entraînement (31) est dans la position d'entraînement.

4. Système de convoyage (3) selon l'une quelconque des revendications 1 à 3, dans lequel le chariot de convoyage autopropulsé (25) comporte au moins une roue motrice (27), et au moins un mécanisme d'entraînement en rotation (28) configuré pour entraîner en rotation l'au moins une roue motrice (27).

5. Système de convoyage (3) selon l'une quelconque des revendications 1 à 4, lequel comporte un élément de guidage de chariot (24) définissant le chemin de convoyage, l'élément de guidage de chariot (24) étant configuré pour recevoir et guider le chariot de convoyage autopropulsé (25) lors des déplacements du chariot de convoyage autopropulsé (25) le long du chemin de convoyage.

6. Système de convoyage (3) selon la revendication 5, dans lequel le chariot de convoyage autopropulsé (25) comporte des galets de guidage (35a, 35b) configurés pour coopérer avec l'élément de guidage de chariot (24) lors des déplacements du chariot de convoyage autopropulsé (25) le long du chemin de convoyage.

7. Système de convoyage (3) selon l'une quelconque des revendications 1 à 6, lequel comprend au moins une zone de prélèvement ou de transfert (36) disposée le long du chemin de guidage et à l'extérieur du chemin de guidage, et dans lequel le chariot de convoyage autopropulsé (25) est configuré pour déplacer le support de récipients (4), reçu sur l'élément de guidage de support (19), dans l'au moins une zone de prélèvement ou de transfert (36) de manière à libérer le chemin de guidage.

8. Système de convoyage (3) selon la revendication 7, dans lequel l'au moins une zone de prélèvement ou de transfert (36) comporte un emplacement de prélèvement agencé pour recevoir et stocker au moins temporairement le support de récipients (4).

9. Système de convoyage (3) selon l'une quelconque des revendications 1 à 8, dans lequel le chariot de convoyage autopropulsé (25) comporte un corps de chariot (26) et un élément de support (29) sur lequel est monté mobile l'élément d'entraînement (31), l'élément de support (29) étant monté mobile en translation par rapport au corps de chariot (26) selon une direction de déplacement transversale au chemin de convoyage et entre au moins une position de convoyage et une position de dégagement.

10. Système de convoyage (3) selon la revendication 9, dans lequel l'élément de support (29) comporte une surface de poussée (29a, 29b) configurée pour exercer un effort de poussée contre le support de récipients (4) lorsque le support de récipients (4) est reçu sur l'élément de guidage de support (19) et l'élément de support (29) est déplacé vers la position de dégagement.

11. Système de convoyage (3) selon l'une quelconque des revendications 1 à 10, lequel comprend une zone de chargement (17) destinée à stocker le support de récipients (4) et comprenant un dispositif de chargement agencé pour charger le support de récipients (4) dans le chemin de guidage défini par l'élément de guidage de support (19), et une zone de déchargement (18) dans laquelle le support de récipients (4) est destiné à être déchargé.

12. Système de convoyage (3) selon l'une quelconque des revendications 1 à 11, lequel comporte une unité de commande (65) configurée pour communiquer à distance avec le chariot de convoyage autopropulsé (25).

13. Système de convoyage selon l'une quelconque des revendications 1 à 12, lequel comporte un rotor de stockage (54) d'axe de rotation sensiblement vertical, le rotor de stockage (54) comportant une pluralité de logements de stockage (55) configurés chacun pour recevoir un support de récipients (4) provenant du chemin de guidage.

14. Système de convoyage (3) selon l'une quelconque des revendications 1 à 13, lequel comporte un premier et un deuxième éléments de guidage de support (19, 21) définissant respectivement un premier et un deuxième chemins de guidage, les premier et deuxième éléments de guidage de support (19, 21) étant disposés de part et d'autre du chemin de convoyage, l'élément d'entraînement (31) du chariot de convoyage autopropulsé (25) étant monté mobile entre une première position d'entraînement dans laquelle l'élément d'entraînement (31) est configuré pour transmettre un mouvement d'entraînement à un support de récipients (4) reçu sur le premier élément de guidage de support (19), une deuxième position d'entraînement dans laquelle l'élément d'entraînement (31) est configuré pour transmettre un mouvement d'entraînement à un support de récipients (4) reçu sur le deuxième élément de guidage de support (21), et une position de libération dans laquelle l'élément d'entraînement (31) est configuré pour libérer lesdits supports de récipients.

15. Système de convoyage (3) selon la revendication 14, lequel comporte un dispositif de transfert (57) configuré pour transférer un support de récipients (4) du premier élément de guidage de support (19) au deuxième élément de guidage de support (21), et inversement.

16. Système d'analyse automatique (2) pour diagnostic in vitro, comprenant un système de convoyage (3) selon l'une quelconque des revendications 1 à 15, et au moins un poste de traitement d'échantillons, tel qu'un poste d'analyse et/ou de mesure (5), disposé le long du chemin de guidage.

## Patentansprüche

1. Beförderungssystem (3), konfiguriert zum Befördern von Gefäßhalterungen (4), dafür geeignet, Gefäße (6) zu halten, die Proben von biologischer Flüssigkeit enthalten, wobei das Beförderungssystem (3) wenigstens umfasst:
- ein Halterungs-Führungselement (19), das einen Führungsweg definiert, wobei das Halterungs-Führungselement (19) dafür konfiguriert ist, eine Gefäßhalterung (4) aufzunehmen und die Gefäßhalterung (4) in Translation an dem Führungsweg entlangzuführen,
- einen selbst-angetriebenen Beförderungswagen (25), fortbewegbar an einem Führungsweg entlang, der sich entlang des Halterungs-Führungselements (19) erstreckt, wobei der selbst-angetriebene Beförderungswagen (25) ein Antriebselement (31) umfasst, beweglich montiert zwischen wenigstens einer Antriebsposition, in der das Antriebselement (31) dafür konfiguriert ist, eine Antriebsbewegung an die Gefäßhalterung (4) zu übertragen, die auf dem Halterungs-Führungselement (19) aufgenommen wird, und einer Freigabeposition, in der das Antriebselement (31) dafür konfiguriert ist, die Gefäßhalterung (4) freizugeben, wobei der selbst-angetriebene Beförderungswagen (25) dafür konfiguriert ist, die Gefäßhalterung (4) in Translation an dem Führungsweg entlang fortzubewegen, wenn sich das Antriebselement (31) in der Antriebsposition befindet und sich der selbst-angetriebene Beförderungswagen (25) an dem Beförderungsweg entlang fortbewegt.

2. Beförderungssystem (3) nach Anspruch 1, wobei das Antriebselement (31) drehbar um eine Drehachse herum montiert ist.

3. Beförderungssystem (3) nach Anspruch 1 oder 2, wobei das Antriebselement (31) zwei Antriebsstränge (32) umfasst, die voneinander beabstandet und dafür konfiguriert sind, mit der Gefäßhalterung (4) zusammenzuwirken, wenn sich das Antriebselement (31) in der Antriebsposition befindet.

4. Beförderungssystem (3) nach einem der Ansprüche 1 bis 3, wobei der selbst-angetriebene Beförderungswagen (25) wenigstens ein Antriebsrad (27) und wenigstens einen rotierenden Antriebsmechanismus (28) umfasst, dafür konfiguriert, das wenigstens eine Antriebsrad (27) in Rotation anzutreiben.

5. Beförderungssystem (3) nach einem der Ansprüche 1 bis 4, das ein Wagenführungselement (24) umfasst, das den Beförderungsweg definiert, wobei das Wagenführungselement (24) dafür konfiguriert ist, den selbst-angetriebenen Beförderungswagen (25) bei den Fortbewegungen des selbst-angetriebenen Beförderungswagen (25) an dem Beförderungsweg entlang aufzunehmen und zu führen.

6. Beförderungssystem (3) nach Anspruch 5, wobei der selbst-angetriebene Beförderungswagen (25) Führungsrollen (35a, 35b) umfasst, dafür konfiguriert, bei den Fortbewegungen des selbst-angetriebenen Beförderungswagen (25) an dem Beförderungsweg entlang mit dem Wagenführungselement (24) zusammenzuwirken.

7. Beförderungssystem (3) nach einem der Ansprüche 1 bis 6, das wenigstens einen Entnahme- oder Transportbereich (36) umfasst, angeordnet an dem Führungsweg entlang und außerhalb des Führungsweges, und wobei der selbst-angetriebene Beförderungswagen (25) dafür konfiguriert ist, in dem wenigstens einen Entnahme- oder Transportbereich (36) die Gefäßhalterung (4), aufgenommen auf dem Halterungs-Führungselement (19), fortzubewegen, sodass der Führungsweg freigegeben wird.

8. Beförderungssystem (3) nach Anspruch 7, wobei der wenigstens eine Entnahme- oder Transportbereich (36) eine Entnahmestelle umfasst, die dafür ausgelegt ist, die Gefäßhalterung (4) aufzunehmen und wenigstens temporär aufzubewahren.

9. Beförderungssystem (3) nach einem der Ansprüche 1 bis 8, wobei der selbst-angetriebene Beförderungswagen (25) einen Wagenkörper (26) und ein Halterungselement (29) umfasst, auf dem das Antriebselement (31) beweglich montiert ist, wobei das Halterungselement (29) beweglich in Translation in Bezug auf den Wagenkörper (26) montiert ist, gemäß einer quer zu dem Beförderungsweg verlaufenden Fortbewegungsrichtung und zwischen wenigstens einer Beförderungsposition und einer Lösungsposition.

10. Beförderungssystem (3) nach Anspruch 9, wobei das Halterungselement (29) eine Schuboberfläche (29a, 29b) umfasst, dafür konfiguriert, eine Schubkraft gegen die Gefäßhalterung (4) auszuüben, wenn die Gefäßhalterung (4) auf dem Halterungs-Führungselement (19) aufgenommen wird und das Halterungselement (29) zu der Lösungsposition fortbewegt wird.

11. Beförderungssystem (3) nach einem der Ansprüche 1 bis 10, das einen Ladebereich (17) umfasst, dafür bestimmt, die Gefäßhalterung (4) aufzunehmen und umfassend eine Ladevorrichtung, dafür ausgelegt, die Gefäßhalterung (4) in dem Führungsweg, definiert durch das Halterungs-Führungselement (19), zu laden, und einen Entladebereich (18), in dem die Gefäßhalterung (4) dafür bestimmt ist, entladen zu werden.

12. Beförderungssystem (3) nach einem der Ansprüche 1 bis 11, das eine Steuereinheit (65) umfasst, dafür konfiguriert, aus der Ferne mit dem selbst-angetriebenen Beförderungswagen (25) zu kommunizieren.

13. Beförderungssystem nach einem der Ansprüche 1 bis 12, das einen Lagerungsrotor (54) mit im Wesentlichen vertikaler Rotationsachse umfasst, wobei der Lagerungsrotor (54) eine Vielzahl von Lagerungsgehäusen (55) umfasst, jedes dafür konfiguriert, eine von dem Führungsweg kommende Gefäßhalterung (4) aufzunehmen.

14. Beförderungssystem (3) nach einem der Ansprüche 1 bis 13, das ein erstes und ein zweites Halterungs-Führungselement (19, 21) umfasst, jeweils einen ersten und einen zweiten Führungsweg definierend, wobei das erste und zweite Halterungs-Führungselement (19, 21) beidseitig des Beförderungsweges angeordnet sind, wobei das Antriebselement (31) des selbst-angetriebenen Beförderungswagens (25) beweglich angeordnet ist zwischen einer ersten Antriebsposition, in der das Antriebselement (31) dafür konfiguriert ist, eine Antriebsbewegung an eine Gefäßhalterung (4), aufgenommen auf dem ersten Halterungs-Führungselement (19), zu übertragen, einer zweiten Antriebsposition, in der das Antriebselement (31) dafür konfiguriert ist, eine Antriebsbewegung an eine Gefäßhalterung (4), aufgenommen auf dem zweiten Halterungs-Führungselement (21), zu übertragen, und einer Freigabeposition, in der das Antriebselement (31) dafür konfiguriert ist, die Gefäßhalterungen freizugeben.

15. Beförderungssystem (3) nach Anspruch 14, das eine Transportvorrichtung (57) umfasst, die dafür konfiguriert ist, eine Gefäßhalterung (4) von dem ersten Halterungs-Führungselement (19) zu dem zweiten Halterungs-Führungselement (21) zu transportieren und umgekehrt.

16. System zur automatischen Analyse (2) für In-Vitro-Diagnose, umfassend ein Beförderungssystem (3) nach einem der Ansprüche 1 bis 15, und wenigstens einen Proben-Behandlungsposten sowie einen Posten zur Analyse und/oder zur Messung (5), angeordnet an dem Führungsweg entlang.

## Claims

1. A conveying system (3) configured to convey containers supports (4) intended to support containers (6) containing samples of a biological liquid, the conveying system (3) including at least:
- a support guide element (19) defining a guide track, the support guide element (19) being configured to receive a containers support (4) and guide said containers support (4) in translation along the guide track,
- a self-propelled conveying carriage (25) displaceable along a conveying track extending along the support guide element (19), the self-propelled conveying carriage (25) comprising a drive element (31) movably mounted between at least a drive position in which the drive element (31) is configured to transmit a drive movement to the containers support (4) received on the support guide element (19), and a release position in which the drive element (31) is configured to release the containers support (4), the self-propelled conveying carriage (25) being configured to displace the containers support (4) in translation along the guide track when the drive element (31) is in the drive position and the self-propelled conveying carriage (25) is displaced along the conveying track.

2. The conveying system (3) according to claim 1, wherein the drive element (31) is pivotally mounted about a pivot axis.

3. The conveying system (3) according to claim 1 or 2, wherein the drive element (31) includes two drive branches (32) spaced from each other and configured to cooperate with the containers support (4) when the drive element (31) is in the drive position.

4. The conveying system (3) according to any one of claims 1 to 3, wherein the self-propelled conveying carriage (25) includes at least one drive wheel (27), and at least one rotational drive mechanism (28) configured to drive the at least one drive wheel (27) in rotation.

5. The conveying system (3) according to any one of claims 1 to 4, which includes a carriage guide element (24) defining the conveying track, the carriage guide element (24) being configured to receive and guide the self-propelled conveying carriage (25) during the displacements of the self-propelled conveying carriage (25) along the conveying track.

6. The conveying system (3) according to claim 5, wherein the self-propelled conveying carriage (25) includes guide rollers (35a, 35b) configured to cooperate with the carriage guide element (24) during the displacements of the self-propelled conveying carriage (25) along the conveying track.

7. The conveying system (3) according to any one of claims 1 to 6, which comprises at least one sampling or transfer area (36) disposed along the guide track and outside the guide track, and wherein the self-propelled conveying carriage (25) is configured to displace the containers support (4), received on the support guide element (19), into the at least one sampling or transfer area (36) so as to release the guide track.

8. The conveying system (3) according to claim 7, wherein the at least one sampling or transfer area (36) includes a sampling location arranged to receive and store at least temporarily the containers support (4).

9. The conveying system (3) according to any one of claims 1 to 8, wherein the self-propelled conveying carriage (25) includes a carriage body (26) and a support element (29) on which the drive element (31) is movably mounted, the support element (29) being mounted movable in translation relative to the carriage body (26) according to a direction of displacement transverse to the conveying track and between at least a conveying position and a clearance position.

10. The conveying system (3) according to claim 9, wherein the support element (29) includes a pushing surface (29a, 29b) configured to exert a pushing force against the containers support (4) when the containers support (4) is received on the support guide element (19) and the support element (29) is displaced toward the clearance position.

11. The conveying system (3) according to any one of claims 1 to 10, which comprises a loading area (17) intended to store the containers support (4) and comprising a loading device arranged to load the containers support (4) in the guide track defined by the support guide element (19), and an unloading area (18) in which the containers support (4) is intended to be unloaded.

12. The conveying system (3) according to any one of claims 1 to 11, which includes a control unit (65) configured to remotely communicate with the self-propelled conveying carriage (25).

13. The conveying system according to any one of claims 1 to 12, which includes a storage rotor (54) with a substantially vertical axis of rotation, the storage rotor (54) including a plurality of storage housings (55) each configured to receive a containers support (4) coming from the guide track.

14. The conveying system (3) according to any one of claims 1 to 13, which includes first and second support guide elements (19, 21) respectively defining first and second guide tracks, the first and second support guide elements (19, 21) being disposed on either side of the conveying track, the drive element (31) of the self-propelled conveying carriage (25) being movably mounted between a first drive position in which the drive element (31) is configured to transmit a drive movement to a containers support (4) received on the first support guide element (19), a second drive position in which the drive element (31) is configured to transmit a drive movement to a containers support (4) received on the second support guide element (21), and a release position in which the drive element (31) is configured to release said containers supports.

15. The conveying system (3) according to claim 14, which includes a transfer device (57) configured to transfer a containers support (4) from the first support guide element (19) into the second support guide element (21), and vice versa.

16. An automatic analysis system (2) for in vitro diagnosis, comprising a conveying system (3) according to any one of claims 1 to 15, and at least one samples processing station, such as an analysis and/or measurement station (5), disposed along the guide track.
